(19) Europäisches Patentamt
European Patent Office
Office européen des brevets

(11) EP 0 741 132 B1

(12) **EUROPEAN PATENT SPECIFICATION**

(45) Date of publication and mention of the grant of the patent:
**10.04.2002 Bulletin 2002/15**

(21) Application number: **95905756.3**

(22) Date of filing: **11.01.1995**

(51) Int Cl.⁷: **C07D 209/42**, A61K 31/40

(86) International application number:
**PCT/JP95/00019**

(87) International publication number:
**WO 95/19343 (20.07.1995 Gazette 1995/31)**

(54) **INDOLE DERIVATIVES**

INDOL-DERIVATE

DERIVES DE L'INDOLE

(84) Designated Contracting States:
**AT BE CH DE DK ES FR GB GR IE IT LI LU MC NL PT SE**

(30) Priority: **18.01.1994 JP 333494**

(43) Date of publication of application:
**06.11.1996 Bulletin 1996/45**

(73) Proprietor: **KYOWA HAKKO KOGYO CO., LTD.**
**Chiyoda-ku, Tokyo 100 (JP)**

(72) Inventors:
- **MACHII, Daisuke**
**Sunto-gun Shizuoka 411 (JP)**
- **TAKAI, Haruki**
**Sunto-gun Shizuoka 411 (JP)**
- **KOSAKA, Nobuo**
**Sunto-gun Shizuoka 411 (JP)**
- **SEO, Hisakatsu**
**Tokyo 156 (JP)**
- **SUGIYAMA, Tomomi**
**Shizuoka 410 (JP)**
- **NAKAMURA, Joji**
**Sunto-gun Shizuoka 411 (JP)**
- **ISHIDA, Hiroyuki**
**Sunto-gun Shizuoka 411 (JP)**
- **GOMI, Katsushige**
**Shizuoka 410-11 (JP)**
- **SATO, Soichiro**
**Sunto-gun Shizuoka 411 (JP)**
- **UCHII, Masako**
**Shizuoka 411 (JP)**

(74) Representative: **VOSSIUS & PARTNER**
**Siebertstrasse 4**
**81675 München (DE)**

(56) References cited:
**EP-A- 0 395 093**
**EP-A- 0 449 196**
**JP-A- 3 215 461**
**JP-A- 4 211 651**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 741 132 B1

## Description

Technical Field

The present invention relates to indole derivatives which are useful as therapeutic agents for osteoporosis.

Background Art

It is disclosed in Japanese Published Unexamined Patent Application No. 215461/91 that a triphenylmethane derivative having a substituent such as carbamoyl is useful as a therapeutic agent for osteoporosis. Further, it is disclosed in Japanese Published Unexamined Patent Application No. 211651/92 that an indole derivative having a phenyl group is useful as a therapeutic agent for osteoporosis.

Disclosure of the Invention

The present invention relates to indole derivatives represented by formula (I):

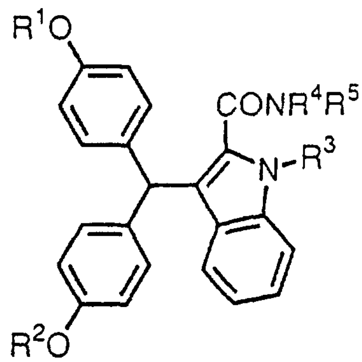

(I)

wherein $R^1$ and $R^2$ independently represent hydrogen, $C_1$-$C_8$ alkyl, $-(CH_2)_n-OR^6$ (wherein $R^6$ represents hydrogen or $C_1$-$C_8$ alkyl, and n is an integer of 1 to 6),
$R^3$ represents hydrogen, $C_1$-$C_8$ alkyl, or

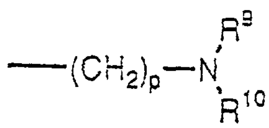

(wherein $R^9$ and $R^{10}$ independently represent hydrogen or $C_1$-$C_8$ alkyl, or $R^9$ and $R^{10}$ are combined together with the adjacent nitrogen atom to form alicyclic heterocyclic group selected from the group consisting of pyrrolidinyl, imidazolidinyl, pyrazolidinyl, piperidino, piperazinyl, homopiperazinyl, morpholino and thiomorpholino (said alicyclic heterocyclic group is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di($C_1$-$C_8$ alkyl)amino, trifluoromethyl, phenyl (said phenyl is optionally substituted with 1 to 3 substituents- selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di($C_1$-$C_8$ alkyl)amino and trifluoromethyl), pyridyl and pyrimidinyl, and p represents an integer of 2 to 6), $R^4$ and $R^5$ independently represent hydrogen, $C_1$-$C_8$ alkyl, $C_3$-$C_8$ alicyclic alkyl, phenyl or naphthyl (said phenyl or naphthyl is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di($C_1$-$C_8$ alkyl)amino, trifluoromethyl, phenyl (said phenyl is optionally subsituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di($C_1$-$C_8$ alkyl)amino and trifluoromethyl), pyridyl and pyrimidinyl or a heterocyclic group selected from the group consisting of pyrrolidinyl, imidazolidinyl, pyrazolidinyl, piperidyl, piperidino, piperazinyl, homopiperazinyl, morpholino and thiomorpholino (said heterocyclic group is optionally substituted with 1

to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di ($C_1$-$C_8$ alkyl)amino, trifluoromethyl, phenyl (said phenyl is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di($C_1$-$C_8$ alkyl)amino and trifluoromethyl), pyridyl and pyrimidinyl), or $R^4$ and $R^5$ are combined together with the adjacent nitrogen atom to form an alicyclic heterocyclic group selected from the group consisting of pyrrolidinyl, imidazolidinyl, pyrazolidinyl, piperidino, piperazinyl, homopiperazinyl, morpholino and thiomorpholino (said alicyclic heterocyclic group is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di($C_1$-$C_8$ alkyl)amino, trifluoromethyl, phenyl (said phenyl is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di ($C_1$-$C_8$ alkyl)amino and trifluoromethyl), pyridyl and pyrimidinyl, or pharmaceutically acceptable salts thereof.

The compounds represented by formula (I) are hereinafter referred to as Compounds (I). The same applies to the compounds of other formula numbers.

In the definitions of the group in the formula (I), the $C_1$-$C_8$ alkyl means a straight or branched alkyl group having 1 to 8 carbon atoms such as methyl, ethyl, propyl, isopropyl, butyl, isobutyl, sec-butyl, tert-butyl, pentyl, neopentyl, hexyl, heptyl, and octyl. The alicyclic alkyl means an alicyclic alkyl group having 3 to 8 carbon atoms such as cyclopropyl, cyclobutyl, cyclopentyl, cyclohexyl, cycloheptyl, and cyclooctyl. The alicyclic heterocyclic group means a group such as pyrrolidinyl, imidazolidinyl, pyrazolidinyl, piperidino, piperazinyl, homopiperazinyl, morpholino, and thiomorpholino.

The substituted alicyclic heterocyclic group, the substituted heterocyclic group, and the substituted aryl each has 1 to 3 independently selected substituents. Examples of the substituents are $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di($C_1$-$C_8$ alkyl)amino, trifluromethyl, substituted or unsubstituted phenyl, pyridyl, and pyrimidinyl.

In the definitions of the substituents, the $C_1$-$C_8$ alkyl and the $C_1$-$C_8$ alkyl moiety of the $C_1$-$C_8$ alkoxy, $C_1$-$C_8$ alkylthio, $C_2$-$C_9$ alkoxycarbonyl, and mono- or di ($C_1$-$C_8$ alkyl)amino have the same meanings as defined for the above-mentioned $C_1$-$C_8$ alkyl. The $C_7$-$C_{13}$ aralkyl means an aralkyl group having 7 to 13 carbon atoms such as benzyl, phenethyl, benzhydryl, and naphthylmethyl, the $C_1$-$C_7$ alkanoyl means an alkanoyl group having 1 to 7 carbon atoms such as formyl, acetyl, propionyl, butyryl, isobutyryl, valeryl, pivaloyl, hexanoyl, and heptanoyl. The halogen includes fluorine, chlorine, bromine, and iodine. The substituted phenyl has 1 to 3 independently selected substituents such as $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di($C_1$-$C_8$ alkyl)amino, trifluoromethyl. The $C_1$-$C_8$ alkyl, $C_1$-$C_8$ alkoxy, $C_1$-$C_8$ alkylthio, $C_7$-$C_{13}$ aralkyl, $C_2$-$C_9$ alkoxy carbonyl, $C_1$-$C_7$ alkanoyl, halogen, and mono- or di($C_1$-$C_8$ alkyl) amino have the same definitions as defined above.

The pharmaceutically acceptable salts of Compounds (I) include inorganic acid addition salts such as hydrochloride, sulfate, nitrate, and phosphate, organic acid addition salts such as acetate, maleate, fumarate, tartrate, citrate, lactate, glyoxylate, aspartate, methane sulfonate, ethane sulfonate, and benzene sulfonate, metal salts such as sodium salt, potassium salt, and calcium salt, ammonium salt, tetramethylammonium salt, and amine addition salts such as a salt with morpholine.

The processes for producing Compounds (I) are described below.

Compound (I) can be prepared according to the following reaction step:

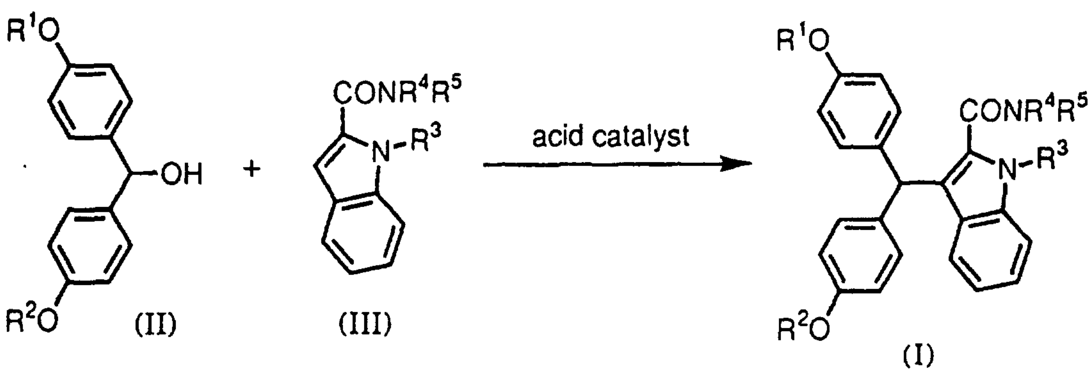

(In the formulae, $R^1$, $R^2$, $R^3$, $R^4$, and $R^5$ have the same meanings as defined above.)

Compound (I) can be obtained by reacting Compound (II) with Compound (III) in the presence of 0.1 to 1 equivalence of an acid catalyst such as boron trifluoride-ether complex, paratoluenesulfonic acid, and trifluoroacetic acid, in a solvent such as methylene chloride, chloroform, ether, and tetrahydrofuran, at a temperature of -20°C to the boiling point of the solvent for 0.5 to 24 hours.

Compound (Ib), which is Compound (I) in which all of $R^1$, $R^2$, and $R^3$ are groups other than hydrogen, can also be prepared from Compound (Ia), which is Compound (I) in which $R^1$ and $R^2$ are groups other than hydrogen, and $R^3$ is hydrogen, according to the following reaction step:

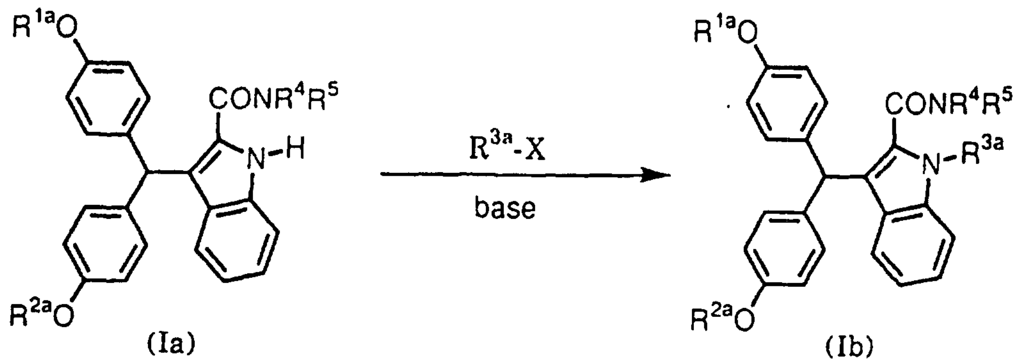


(Ia) (Ib)

(In the formulae, $R^{1a}$, $R^{2a}$, and $R^{3a}$ each represents a group other than hydrogen in the definition of $R^1$, $R^2$, and $R^3$, X represents chlorine, bromine, or iodine, and $R^4$ and $R^5$ have the same meanings as defined above.)

Compound (Ib) can be obtained by reacting Compound (Ia) with a halogenated alkyl or halogenated aminoalkyl in the presence of a base such as sodium hydride and potassium tert-butoxide, in a solvent such as a lower alcohol (e.g., methanol, ethanol), N,N-dimethylformamide, dimethyl sulfoxide, and tetrahydrofuran, at a temperature of 0°C to the boiling point of the solvent for 0.5 to 24 hours.

Compound (Iba), which is Compound (Ib) in which $R^{3a}$ is

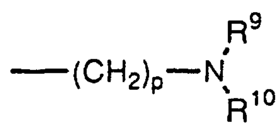

(wherein $R^9$, $R^{10}$, and p have the same meanings as defined above), can be prepared from Compound (Ia) according to the following reaction steps:

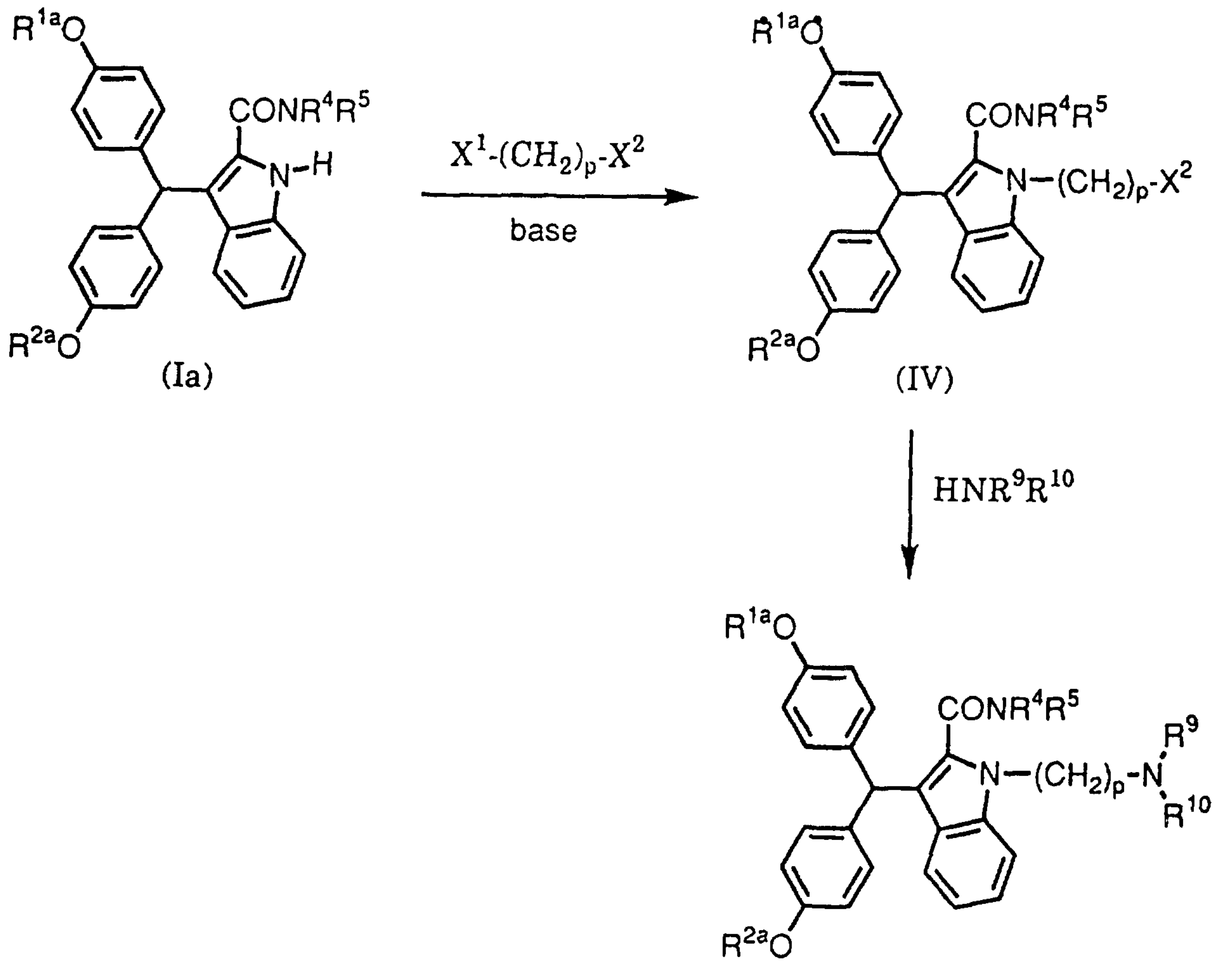


(Ia)

(IV)

(Iba)

(In the formulae, $X^1$ and $X^2$ independently represent chlorine, bromine, or iodine, and $R^{1a}$, $R^{2a}$, $R^4$, $R^5$, $R^9$, $R^{10}$, and p have the same meanings as defined above.)

Compound (IV) can be obtained by reacting Compound (Ia) with α,ω-dihalogenated alkyl in the presence of a base such as sodium hydride and potassium tert-butoxide, in a solvent such as a lower alcohol (e.g., methanol, ethanol), N,N-dimethylformamide, dimethyl sulfoxide, and tetrahydrofuran, at a temperature of 0°C to the boiling point of the solvent for 0.5 to 24 hours. Then, Compound (Iba) can be obtained by reacting Compound (IV) with 1 equivalence to an excess amount of ammonia, a primary amine, or a secondary amine, in a solvent such as a lower alcohol (e.g., methanol, ethanol), N,N-dimethylformamide, dimethyl sulfoxide, tetrahydrofuran, and water, or a mixed solvent thereof, if necessary, in the presence of an inorganic base such as potassium carbonate and sodium carbonate, at a temperature of 0°C to the boiling point of the solvent for one hour to 7 days.

Compound (Id), which is Compound (I) in which both of $R^1$ and $R^2$ are hydrogen, can be prepared from Compound (Ic), which is Compound (I) in which both of $R^1$ and $R^2$ are methoxymethyl, according to the following reaction step:

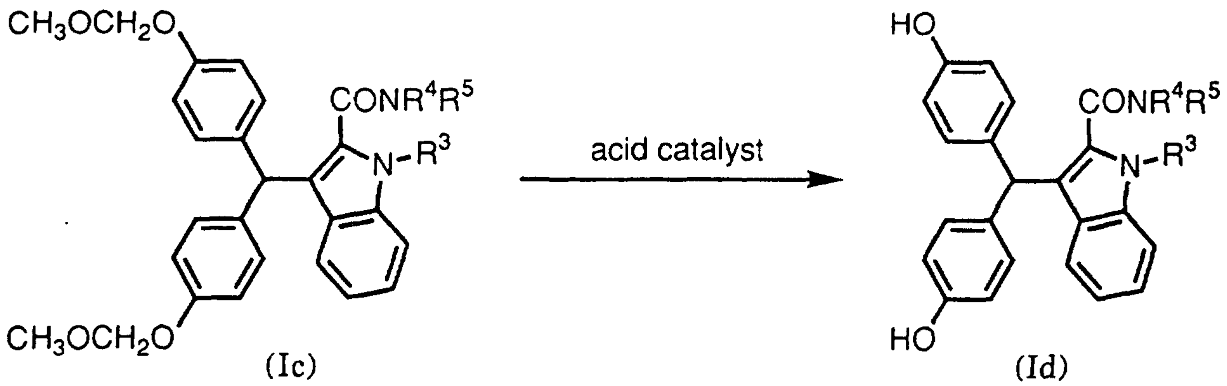


(Ic) (Id)

(In the formulae, $R^3$, $R^4$, and $R^5$ have the same meanings as defined above.)

Compound (Id) can be prepared by treating Compound (Ic) in the presence of an acid catalyst such as hydrochloric acid, sulfuric acid, and paratoluenesulfonic acid, in a solvent such as a lower alcohol (e.g., methanol, ethanol), N,N-dimethylformamide, dimethyl sulfoxide, tetrahydrofuran, and water, or a mixed solvent thereof, at a temperature of room temperature to the boiling point of the solvent for 0.5 to 24 hours.

Compound (I) can also be produced from Compound (V) according to the following reaction step:

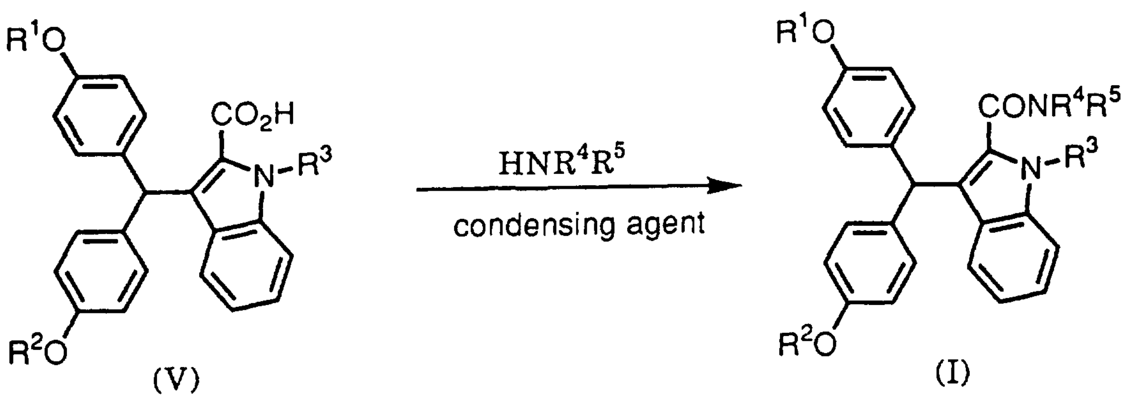


(V) (I)

(In the formulae, $R^1$, $R^2$, $R^3$, $R^4$, and $R^5$ have the same meanings as defined above.)

Compound (I) can be produced by reacting Compound (V) with ammonia, a primary amine, or a secondary amine in the presence of a condensing agent such as dicyclohexylcarbodiimide and 1-(3-dimethylaminopropyl)-3-ethylcarbodiimide hydrochloride, in a solvent such as methylene chloride, chloroform, and tetrahydrofuran, at a temperature of room temperature to the boiling point of the solvent for 1 to 24 hours.

As for the starting compounds, Compound (II) can be obtained according to the method described in Tetrahedron Lett., 28, 5651 (1987) or a similar method thereto, and Compound (III) can be obtained according to the method described in J. Am. Chem. Soc., 67, 423 (1945) or J. Med. Chem.,32, 1681 (1989), or a similar method thereto.

Then, the processes for producing the starting Compounds (V) are described below.

Compound (Va), which is Compound (V) in which all of $R^1$, $R^2$, and $R^3$ are the groups other than hydrogen, can be produced from Compound (IIa) and Compound (1) according to the following reaction steps:

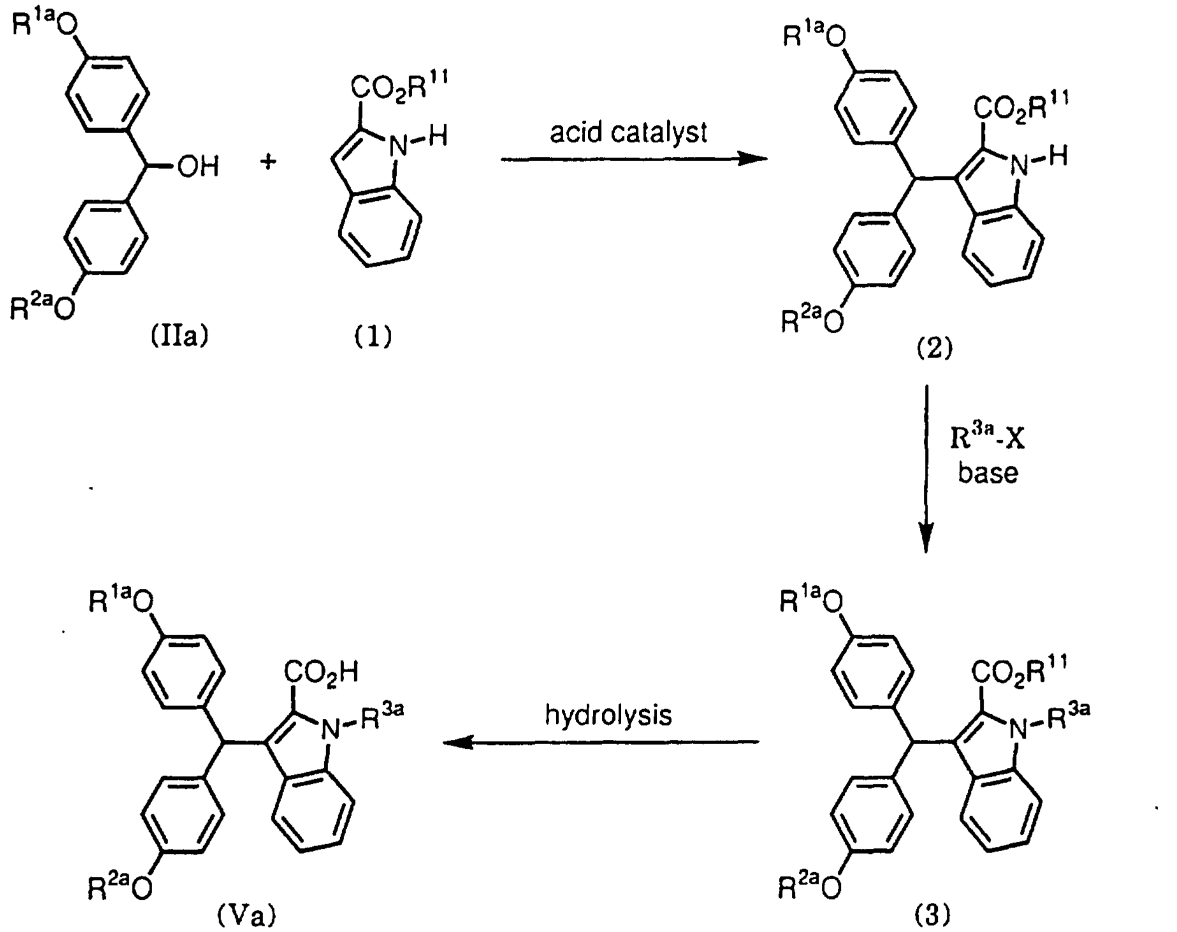


(IIa) (1) (2) (3) (Va)

(In the formulae, $R^{11}$ represents lower alkyl, and X, $R^{1a}$, $R^{2a}$, and $R^{3a}$ have the same meanings as defined above.)

The lower alkyl in the definition of $R^{11}$ has the same meaning as defined above.

Compound (2) can be obtained from Compound (IIa) and Compound (1) which can be obtained by the method described in Chem. Pharm. Bull., 21, 1481 (1973) or a similar method thereto, according to a method similar to that in producing Compound (I) from Compound (II) and Compound (III). Compound (3) can be obtained from Compound (2) according to a method similar to that in producing Compound (Ib) from Compound (Ia). Then, Compound (Va) can be obtained from Compound (3) under the normal hydrolytic condition, for example, treating Compound (3) in the presence of a base such as lithium hydroxide, sodium hydroxide, and potassium hydroxide, in a solvent such as a lower alcohol (e.g., methanol, ethanol), tetrahydrofuran, N,N-dimethylformamide, and dimethyl sulfoxide, if necessary, in the presence of water, at a temperature of room temperature to the boiling point of the solvent for 0.5 to 24 hours.

Compound (Vb), which is Compound (V) in which $R^1$ and $R^2$ are groups other than hydrogen and $R^3$ is hydrogen, can be produced from Compound (2) by hydrolysis of the ester moiety according to the method of producing Compound (Va) from Compound (3) or a similar method thereto.

Compound (Vc), which is Compound (V) in which all of $R^1$, $R^2$, and $R^3$ are hydrogen, or Compound (Vd), which is Compound (V) in which $R^1$ and $R^2$ are hydrogen and $R^3$ is a group other than hydrogen, can be obtained from Compound (2a), which is Compound (2) in which both of $R^{1a}$ and $R^{2a}$ are methoxymethyl, or from Compound (3a), which is Compound (3) in which both of $R^{1a}$ and $R^{2a}$ are methoxymethyl, respectively, by carrying out demethoxymethylation according to the method of producing Compound (Id) from Compound (Ic) or a similar method thereto, followed by hydrolysis of the ester moiety according to the method of producing Compound (Va) from Compound (3) or a similar method thereto.

The intermediates and the desired compounds in the processes described above can be isolated and purified by purification methods conventionally used in organic synthetic chemistry, for example, filtration, extraction, washing, drying, concentration, recrystallization, and various kinds of chromatography. The intermediates may also be subjected to the subsequent reaction without purification.

In the case where a salt of Compound (I) is desired and it is produced in the form of the desired salt, it can be subjected to purification as such. In the case where Compound (I) is produced in the free state and its salt is desired,

Compound (I) is dissolved or suspended in a suitable solvent, followed by addition of an appropriate acid or base to form a salt, and then the salt can be isolated.

Compounds (I) and pharmaceutically acceptable salts thereof may be in the form of adducts with water or various solvents, which are also within the scope of the present invention.

Examples of Compounds (I) obtained in the above processes are shown in Table 1.

Table 1-1

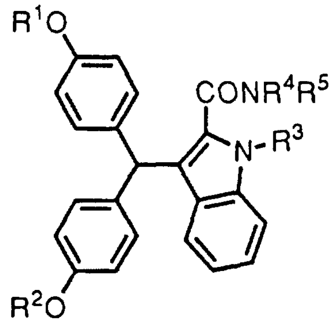

| Compd. No. | $R^1$ | $R^2$ | $R^3$ | $-NR^4R^5$ |
|---|---|---|---|---|
| 1 | $CH_2OCH_3$ | $CH_2OCH_3$ | H | 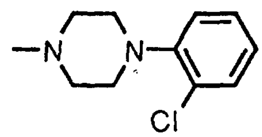 |
| 2 | $CH_2OCH_3$ | $CH_2OCH_3$ | 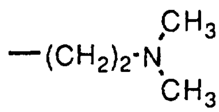 | 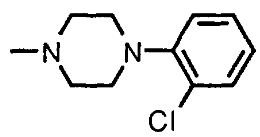 |
| 3 | $CH_2OCH_3$ | $CH_2OCH_3$ | 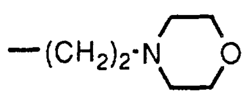 | 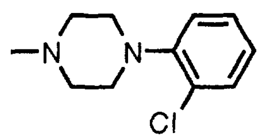 |
| 4 | $CH_2OCH_3$ | $CH_2OCH_3$ | 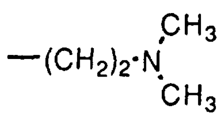 | 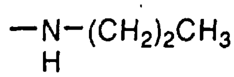 |
| 5 | $CH_2OCH_3$ | $CH_2OCH_3$ | 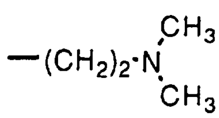 | 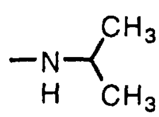 |
| 6 | $CH_2OCH_3$ | $CH_2OCH_3$ | 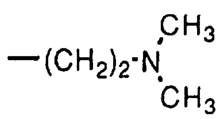 | 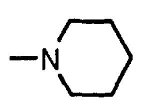 |
| 7 | $CH_2OCH_3$ | $CH_2OCH_3$ | 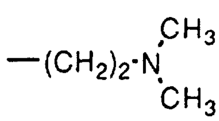 | 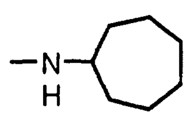 |

Table 1-2

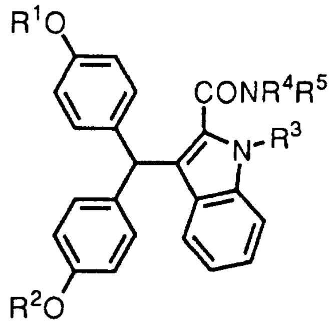

| Compd. No. | $R^1$ | $R^2$ | $R^3$ | $-NR^4R^5$ |
|---|---|---|---|---|
| 8 | H | H | H | 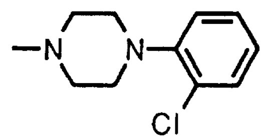 |
| 9 | H | H | 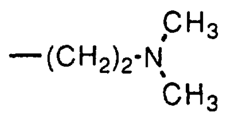 | 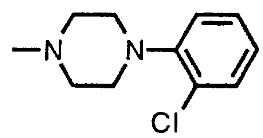 |
| 10 | H | H | 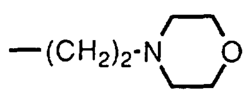 | 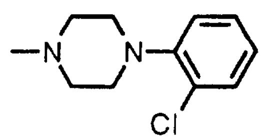 |
| 11 | H | H | 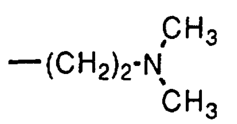 | 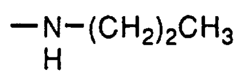 |
| 12 | H | H | 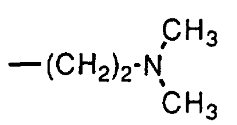 | 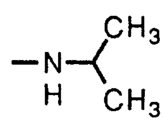 |
| 13 | H | H | 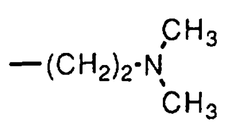 | 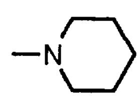 |
| 14 | H | H | 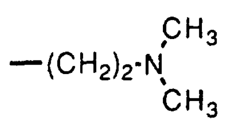 | 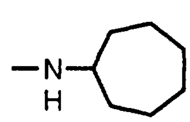 |

## Table 1-3

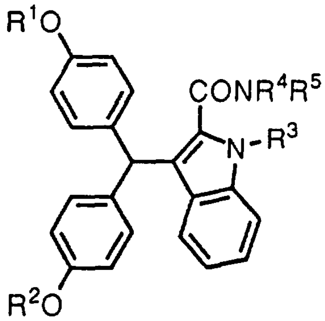

| Compd. No. | $R^1$ | $R^2$ | $R^3$ | $-NR^4R^5$ |
|---|---|---|---|---|
| 15 | $CH_2OCH_3$ | $CH_2OCH_3$ | 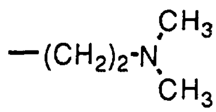 | 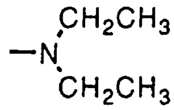 |
| 16 | $CH_2OCH_3$ | $CH_2OCH_3$ | 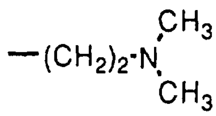 | 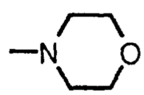 |
| 17 | $CH_2OCH_3$ | $CH_2OCH_3$ | 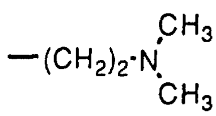 | 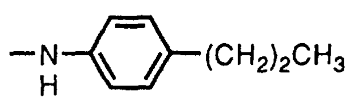 |
| 18 | $CH_2OCH_3$ | $CH_2OCH_3$ | 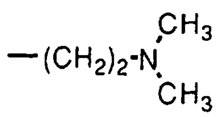 | 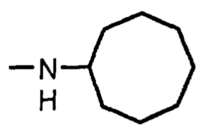 |
| 19 | $CH_2OCH_3$ | $CH_2OCH_3$ | 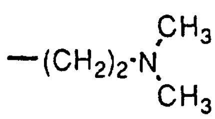 | 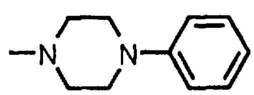 |
| 20 | $CH_2OCH_3$ | $CH_2OCH_3$ | 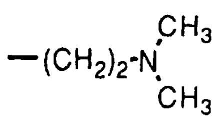 | 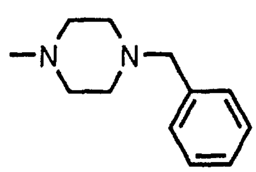 |
| 21 | $CH_2OCH_3$ | $CH_2OCH_3$ | 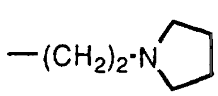 | 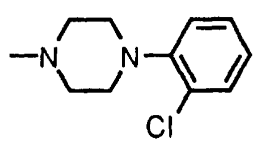 |

Table 1–4

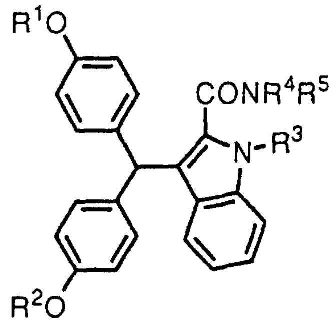

| Compd. No. | $R^1$ | $R^2$ | $R^3$ | $-NR^4R^5$ |
|---|---|---|---|---|
| 22 | $CH_2OCH_3$ | $CH_2OCH_3$ | 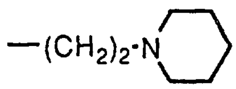 | 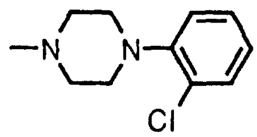 |
| 23 | $CH_2OCH_3$ | $CH_2OCH_3$ | 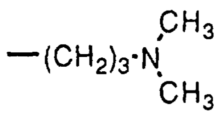 | 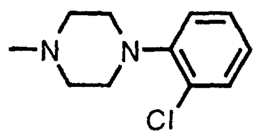 |
| 24 | $CH_2OCH_3$ | $CH_2OCH_3$ | 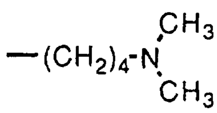 | 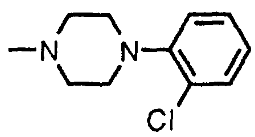 |
| 25 | $CH_2OCH_3$ | $CH_2OCH_3$ | 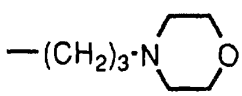 | 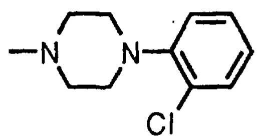 |
| 26 | $CH_2OCH_3$ | $CH_2OCH_3$ | 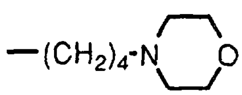 | 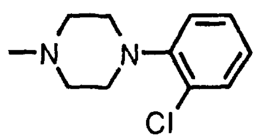 |
| 27 | $CH_2OCH_3$ | $CH_2OCH_3$ | 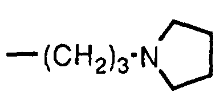 | 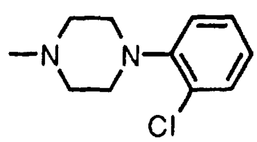 |
| 28 | $CH_2OCH_3$ | $CH_2OCH_3$ | 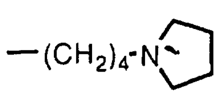 | 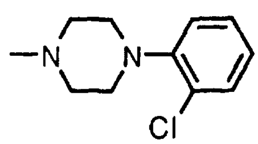 |

Table 1-5

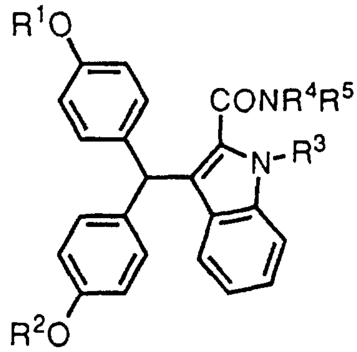

| Compd. No. | $R^1$ | $R^2$ | $R^3$ | $-NR^4R^5$ |
|---|---|---|---|---|
| 29 | $CH_3$ | $CH_3$ | H | 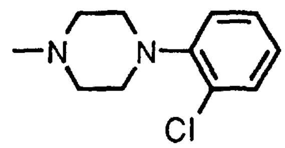 |
| 30 | $CH_3$ | $CH_3$ | 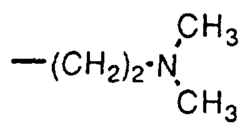 | 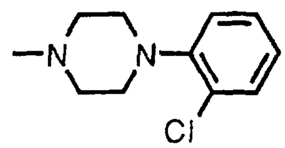 |
| 31 | $CH_3$ | $CH_3$ | 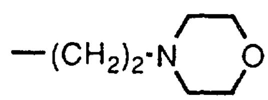 | 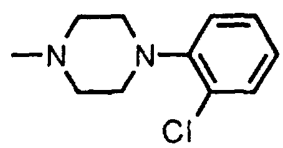 |
| 32 | $CH_3$ | $CH_3$ | 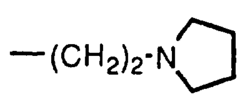 | 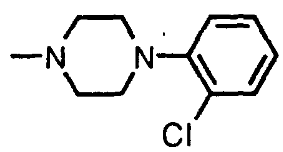 |

Table 1–6

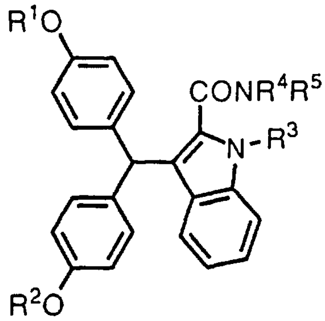

| Compd. No. | $R^1$ | $R^2$ | $R^3$ | $-NR^4R^5$ |
|---|---|---|---|---|
| 33 | H | H | 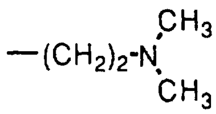 | 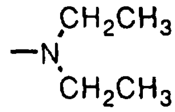 |
| 34 | H | H | 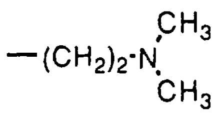 | 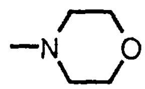 |
| 35 | H | H | 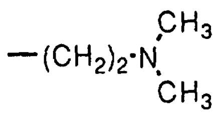 | 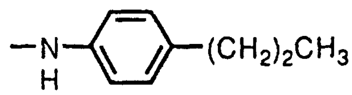 |
| 36 | H | H | 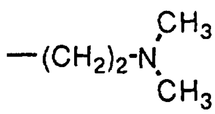 | 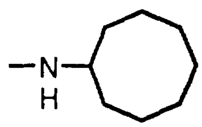 |
| 37 | H | H | 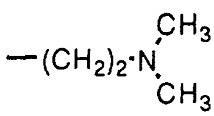 | 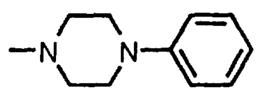 |
| 38 | H | H | 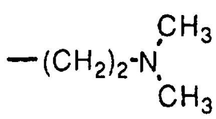 | 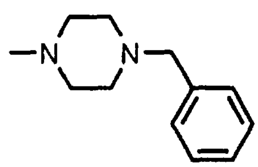 |
| 39 | H | H | 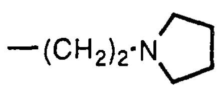 | 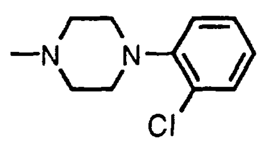 |

Table 1-7

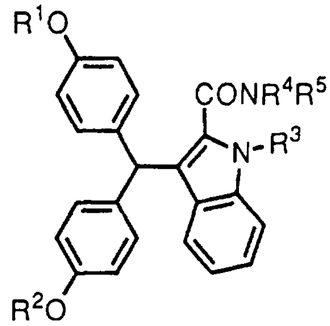

| Compd. No. | $R^1$ | $R^2$ | $R^3$ | $-NR^4R^5$ |
|---|---|---|---|---|
| 40 | H | H | 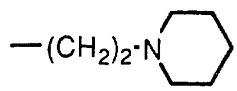 | 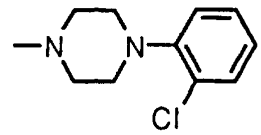 |
| 41 | H | H | 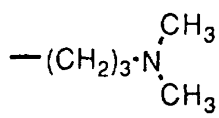 | 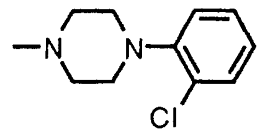 |
| 42 | H | H | 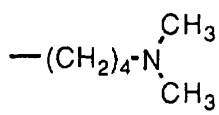 | 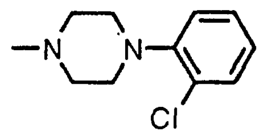 |
| 43 | H | H | 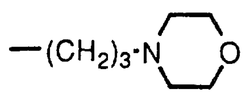 | 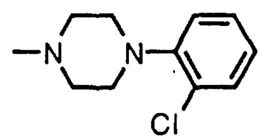 |
| 44 | H | H | 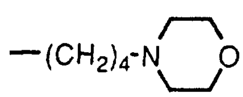 | 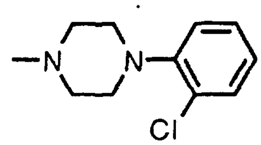 |
| 45 | H | H | 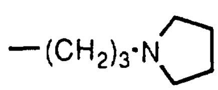 | 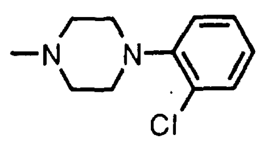 |
| 46 | H | H | 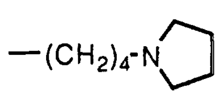 | 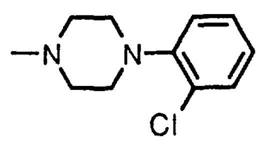 |

The inhibitory effect of the compounds of the present invention on bone absorption is shown below by test examples.

Test Example 1: Inhibitory effect on bone absorption

The calvaria was excised from a newborn dd mouse (5 to 6 days old) under sterile conditions. The calvaria was washed with a modified Dulbecco phosphate buffer physiological saline solution containing neither calcium nor magnesium (a product of Gibco Oriental), and divided into two parts along the center suture. A half of the calvaria was cultivated in a modified Dulbecco Eagle medium (1.5 ml) (a product of Gibco Oriental) containing 2.5% fetal calf serum and 15% equine serum which had been inactivated by heating at 56°C for 20 minutes. The test compound was dissolved in dimethyl sulfoxide and 10 μl of the resulting solution was added to the culture medium to give the final concentrations of 3 x $10^{-6}$M, 1 x $10^{-5}$M, and 3 x $10^{-5}$M. PTH (parathyroid hormone) was dissolved in a 0.15 M saline solution (pH 3) and 3 μl of the resulting solution was added to the culture medium to give the final concentration of 1 x $10^{-8}$M. The culturing was carried out under the condition of 95 % air and 5 % carbon dioxide and at a temperature of 37°C for 96 hours. At 48 hours after the start of the culturing, the culture medium was renewed and the test compound and PTH treated as defined above were added thereto. For examining the effect of the test compound on calcium liberation (bone absorption) from the PTH enhanced bone, a control group, a group using PTH (1 x $10^{-8}$M), and a group using both the test compound (3 x $10^{-6}$M, 1 x $10^{-5}$M, 3 x $10^{-5}$M) and PTH were prepared. The amount of the bone absorption was determined by measuring the amount of calcium accumulated in the culture as collected after 96 hours of culturing. The total calcium concentration in the culture was measured with Calcium C Test Wako. The inhibition rate was calculated using the following equation and the result was shown in terms of 50% inhibitory concentration ($IC_{50}$). The results are shown in Table 2.
Inhibition of bone absorption from PTH enhanced bone

$$\text{Inhibition rate (\%)} = [(C_P - C_D)/(C_P - C_0)] \times 100$$

$C_0$ : The total calcium concentration in the culture containing neither the test compound nor PTH.
$C_P$ : The total calcium concentration in the culture treated with only PTH.
$C_D$ : The total calcium concentration in the culture treated . with both the test compound and PTH.

Table 2

| Compound No. | Inhibitory effect on bone absorption ($IC_{50}$;μM) |
|---|---|
| 8 | >30 |
| 9 | 11.5 |
| 10 | 9.6 |
| 11 | >30 |
| 12 | >30 |
| 13 | 15.0 |
| 14 | 11.9 |

Test Example 2: Inhibitory effect on decrease of bone density by ovariectomy

The experiment was carried out using 10-weeks-old female SD strain rats (Charles River Japan Inc.) The ovaries on both sides of the rats were excised under anesthesia using Nembutal. The test compound (10 mg/kg) was dissolved or suspended in distilled water for injection (a product of Otsuka Pharmaceutical Co., Ltd.) and administered orally once a day from the first day after the operation for 3 weeks. For the control group, distilled water for injection alone was administered. The rat was slaughtered by neck dislocation, the right hindlimb was ablated, and then the bone density of the neck was measured according to a DEXA (DXA) method [Japan Clinic, 52 (9), 2329-2334 (1994)] using bone mineral measuring apparatus DCS-600 (a product of Aloka Co., Ltd.) The inhibition rate of decrease in the bone density of the test compound-administered group was calculated from the decrease of the bone density in the test compound-administered group and that in the control group. The results are shown in Table 3.

Table 3

| Compd No. | Inhibition rate of decrease in bone density (%) |
|---|---|
| 9 | 97 |
| 10 | 33 |

Compounds (I) and pharmaceutically acceptable salts thereof can be formulated into generally employed dose forms such as tablets, capsules, and syrups, and administered orally or parenterally through intramuscular injection, intravenous injection, drip infusion, or rectal administration using suppositories. For preparing these dose forms for oral or parenteral administration, generally known techniques are applied. For example, the preparations may contain various excipients, lubricants, binders, disintegrating agents, isotonizing agents, emulsifiers, and the like.

Examples of the carriers which can be used are water, injectable distilled water, physiological saline, glucose, fructose, sucrose, mannitol, lactose, starch, cellulose, methyl cellulose, carboxymethyl cellulose, hydroxypropyl cellulose, alginic acid, talc, sodium citrate, calcium carbonate, calcium hydrogenphosphate, magnesium stearate, urea, silicone resins, sorbitan fatty acid esters, and glycerin fatty acid esters.

The effective dose and administration schedule of Compound (I) or a pharmaceutically acceptable salt thereof varies depending upon the mode of administration, the age, body weight and conditions of a patient, etc. However, generally, Compound (I) or a pharmaceutically acceptable salt thereof is administered in a dose of 0.1 to 10 mg/kg/day in 1 to 4 parts.

Certain embodiments of the present invention are illustrated in the following Examples and Reference Examples.

## Best Mode for Carrying out the Invention

### Example 1

1-{3-{Bis[4-(methoxymethoxy)phenyl]methyl}indol-2-ylcarbonyl}-4-(2-chlorophenyl)piperazine (Compound 1)

Boron trifluoride-ether complex (0.36 ml, 2.94 mmol) was added dropwise at 0°C to a solution of 4,4'-bis(methoxymethoxy)benzhydrol (8.96 g, 29.43 mmol) and 1-(2-chlorophenyl)-4-(indol-2-ylcarbonyl)piperazine (10.0g, 29.43 mmol) in 200 ml of methylene chloride, followed by stirring at 0°C to room temperature for 2 hours. A saturated aqueous solution of sodium bicarbonate was added to the reaction solution for neutralization followed by extraction with chloroform. The resulting organic layer was washed with a saturated aqueous solution of sodium bicarbonate and a saturated aqueous solution of sodium chloride, and dried over magnesium sulfate, and the solvent was distilled off under reduced pressure to give a crude product. The obtained crude product was recrystallized from ethanol to give 15.1 g (yield: 82%) of the title compound.

$^1$H-NMR($CDCl_3$) δ(ppm): 2.65-2.85 (4H, m), 3.43 (6H, s), 3.45-3.65 (4H, m), 5.11 (4H, s), 5.77 (1H, s), 6.8-6.85 (1H, m), 6.9-7.05 (2H, m), 6.92 (4H, d, J=8.4Hz), 7.1-7.25 (3H, m), 7.17 (4H, d, J=8.4Hz), 7.3-7.35 (2H, m), 8.72 (1H, s).
IR(KBr tab.): 1608, 1509, 1481, 1439, 1233, 1153 $cm^{-1}$
Melting Point : 178.9-180.0°C

### Example 2

1-{3-(Bis[4-(methoxymethoxy)phenyl]methyl}-1-(2-dimethylaminoethyl)indol-2-ylcarbonyl}-4-(2-chlorophenyl)-piperazine (Compound 2)

To a solution of Compound 1 (2.0 g, 3.19 mmol) obtained in Example 1 in 30 ml of N,N-dimethylformamide was portionwise added sodium hydride (60% in oil, 270 mg, 6.71 mmol) with stirring at 0°C, and 2-dimethylaminoethylchloride hydrochloride (460 mg, 3.19 mmol) was added thereto, followed by heating to 80°C and then stirring for one hour. A saturated aqueous solution of ammonium chloride was added to the reaction solution for neutralization, and water was added thereto followed by extraction with ethyl acetate. The resulting organic layer was washed with a saturated aqueous solution of sodium chloride and dried over magnesium sulfate, and the solvent was distilled off under reduced pressure to give 2.9 g of a crude product. The obtained crude product was purified with silica gel column chromatography (ethyl acetate/hexane = 1/1 - ethyl acetate alone) to give 2.1 g (yield: 94%) of the title compound.

$^1$H-NMR ($CDCl_3$) δ(ppm): 2.15-2.4 (2H, m), 2.32 (6H, s), 2.5-2.9 (3H, m), 3.0-3.2 (2H, m), 3.2-3.3 (1H, m), 3.41 (3H, s), 3.43 (3H, s), 3.65-3.8 (1H, m), 3.95-4.2 (2H, m), 4.3-4.45 (1H, m), 5.11 (4H, s), 5.67 (1H, s), 6.75-6.85 (1H, m), 6.85-7.05 (6H, m), 7.05-7.3 (7H, m), 7.3-7.4 (2H, m).
IR(neat) : 1639, 1508, 1459, 1438, 1229, 1153 $cm^{-1}$

Example 3

1-{3-(Bis[4-(methoxymethoxy)phenyl]methyl}-1-(2-morpholinoethyl)indol-2-ylcarbonyl}-4-(2-chlorophenyl)-piperazine (Compound 3)

Substantially the same procedure as in Example 2 was repeated using Compound 1 (2.0 g, 3.19 mmol) obtained in Example 1 and 2-morpholinoethylchloride hydrochloride (600 mg, 3.19 mmol) to give 2.1 g (yield: 89%) of the title compound.

$^1$H-NMR($CDCl_3$) δ(ppm): 2.1-2.25 (1H, m), 2.45-2.7 (6H, m), 2.75-2.95 (2H, m), 3.0-3.15 (2H, m), 3.15-3.3 (1H, m), 3.41 (3H, s), 3.44 (3H, s), 3.6-3.8 (5H, m), 3.9-4.2 (2H, m), 4.25-4.45 (1H, m), 5.11 (2H, s), 5.15 (2H, s), 5.67 (1H, s), 6.75-7.45 (16H, m).
IR(KBr tab.): 1635, 1509, 1432, 1233, 1152, 1004 $cm^{-1}$

Example 4

N-Propyl-3-{bis[4-(methoxymethoxy)phenyl]methyl}-1-(2-dimethylaminoethyl)indole-2-carboxamide (Compound 4)

1-(3-Dimethylaminopropyl)-3-ethylcarbodiimide hydrochloride (728 mg, 3.80 mmol) was added to a solution of 3-{bis[4-(methoxymethoxy)phenyl]methyl}-1-(2-dimethylaminoethyl)indole-2-carboxylic acid (1.0 g, 1.90 mmol) obtained in Reference Example 3 and propylamine (0.23 ml, 2.85 mmol) in 10 ml of methylene chloride, followed by stirring at room temperature for 3.5 hours. Water was added to the reaction solution followed by extraction with chloroform. The resulting organic layer was washed successively with 2N hydrochloric acid, a saturated aqueous solution of sodium bicarbonate, and a saturated aqueous solution of sodium chloride, and dried over magnesium sulfate, and the solvent was distilled off under reduced pressure to give 1.2 g (quantitative) of the title compound.

$^1$H-NMR($CDCl_3$) δ(ppm): 0.86 (3H, t, J=7.4Hz), 1.4-1.6 (2H, m), 2.25 (6H, s), 2.85-2.95 (2H, m), 3.25-3.35 (2H, m), 3.46 (6H, s), 4.44 (2H, t, J=6.6Hz), 5.14 (4H, s), 5.99 (1H, s), 6.85-7.0 (6H, m), 7.10 (4H, d, J=6.6Hz), 7.15-7.25 (1H, m), 7.34 (1H, d, J=8.3Hz).

In the following Examples 5 to 7, substantially the same procedure as in Example 4 was repeated using corresponding amines in place of propylamine to give the desired compounds.

Example 5

N-Isopropyl-3-{bis[4-(methoxymethoxy)phenyl]-methyl}-1-(2-dimethylaminoethyl)indole-2-carboxamide (Compound 5)

$^1$H-NMR($CDCl_3$) δ(ppm): 1.12 (6H, d, J=6.6Hz), 2.24 (6H, s), 2.7-2.8 (2H, br), 3.46 (6H, s), 4.15-4.3 (1H, m), 4.4-4.5 (2H, m), 5.14 (4H, s), 5.93 (1H, s), 6.85-6.95 (6H, m), 7.10 (4H, d, J=8.9Hz), 7.15-7.25 (1H, m), 7.3-7.4 (1H, m).

Example 6

1-{3-{Bis[4-(methoxymethoxy)phenyl]methyl}-1-(2-dimethylaminoethyl)indol-2-ylcarbonyl}piperidine (Compound 6)

$^1$H-NMR($CDCl_3$) δ(ppm): 0.95-1.1 (1H, m), 1.1-1.3 (1H, m), 1.4-1.8 (4H, m), 2.31 (6H, s), 2.55-2.85 (3H, m), 3.0-3.1 (1H, m), 3.4-3.5 (1H, m), 3.44 (3H, s), 3.46 (3H, s), 3.65-3.75 (1H, m), 4.0-4.15 (1H, m), 4.25-4.4 (1H, m), 5.11 (2H, s), 5.14 (2H, s), 5.59 (1H, s), 6.8-7.0 (4H, m), 7.0-7.2 (7H, m), 7.33 (1H, d, J=8.2Hz).

Example 7

N-Cycloheptyl-3-{bis[4-(methoxymethoxy)phenyl]-methyl}-1-(2-dimethylaminoethyl)indole-2-carboxamide (Compound 7)

$^1$H-NMR($CDCl_3$) δ(ppm): 1.2-1.4 (2H, m), 1.4-1.6 (8H, m), 1.9-2.0 (2H, m), 2.23 (6H, s), 2.7-2.8 (2H, m), 3.46 (6H, s), 4.0-4.2 (1H, m), 4.45 (2H, t, J=6.9 Hz), 5.14 (4H, s), 5.94 (1H, s), 6.8-6.95 (6H, m), 7.09 (4H, d, J=8.5Hz), 7.15-7.25 (1H, m), 7.35 (1H, d, J=8.2Hz).

Example 8

1-{3-[Bis(4-hydroxyphenyl)methyl]indol-2-ylcarbonyl}-4-(2-chloroghenyl)piperazine (Compound 8)

Compound 1 (1.43 g, 2.28 mmol) obtained in Example 1 was dissolved in a mixed solvent of tetrahydrofuran (20 ml) and ethanol (20 ml), and 5 ml of 2N hydrochloric acid was added thereto, followed by heating under reflux for 0.5 hours. The solvent was distilled off under reduced pressure, and a saturated aqueous solution of sodium bicarbonate was added thereto followed by extraction with ethyl acetate. The resulting organic layer was washed with a saturated aqueous solution of sodium chloride and dried over magnesium sulfate, and the solvent was distilled off under reduced pressure. The resulting residue was washed with diethyl ether/ethyl acetate and crystallized to give 1.1 g (yield: 88%) of the title compound.

$^1$H-NMR(DMSO-$d_6$) δ(ppm): 2.5-2.9 (2H, m), 3.2-3.6 (6H, m), 5.59 (1H, s), 6.64 (4H, d, J=8.7Hz), 6.8-6.9 (1H, m), 6.98 (4H, d, J=8.7Hz), 7.0-7.15 (4H, m), 7.25-7.45 (3H, m), 9.16 (2H, s), 11.35 (1H, s).
IR(KBr tab.): 1611, 1509, 1477, 1434, 1233 $cm^{-1}$
Melting Point: 226-230°C

Example 9

1-{3-[Bis(4-hydroxyphenyl)methyl]-1-(2-dimethyl aminoethyl) indol-2-ylcarbonyl}-4-(2-chlorophenyl)piperazine methanesulfonate (Compound 9 methanesulfonate)

Compound 2 (13.1 g, 18.4 mmol) obtained in Example 2 was dissolved in a mixed solvent of tetrahydrofuran (50 ml) and ethanol (100 ml), and 20 ml of 2 N hydrochloric acid was added thereto, followed by heating under reflux for 4 hours. The solvent was distilled off under reduced pressure, and a saturated aqueous solution of sodium bicarbonate and ethyl acetate were added thereto for neutralization. At this time, crystals were gradually precipitated. The aqueous layer was separated off and a saturated aqueous solution of sodium chloride was added to the suspending organic layer for washing. The aqueous layer was separated off, and crystals were collected by filtration from the suspending organic layer and dried under reduced pressure to give 8.8 g (yield: 79%) of the free base of the title compound. The crystals were suspended in 500 ml of ethyl acetate, and methanesulfonic acid (0.95 ml, 14.6 mmol) was added thereto, followed by stirring at room temperature. The precipitated crystals were collected by filtration and dried under reduced pressure to give 10.9 g (quantitative) of'the title compound.

$^1$H-NMR(DMSO-$d_6$) δ(ppm): 2.15-2.3 (1H, m), 2.33 (3H, s), 2.5-2.65 (1H, m), 2.7-2.95 (7H, m), 2.95-3.2 (2H, m), 3.3-3.5 (3H, m), 3.6-3.75 (1H, m), 3.8-3.95 (1H, m), 4.2-4.4 (1H, m), 4.5-4.7 (1H, m), 5.49 (1H, s), 6.55-6.7 (4H, m), 6.8-7.1 (8H, m), 7.15-7.35 (2H, m), 7.39 (1H, d, J=7.9 Hz), 7.60 (1H, d, J=8.3Hz), 9.20 (1H, s), 9.27 (1H, s), 9.55-9.7 (1H, br).
IR(KBr tab.): 1609, 1512, 1481, 1442, 1210 $cm^{-1}$
Melting Point: 148-151°C

In the following Examples 10 to 14, substantially the same procedure as in Example 9 was repeated using corresponding Compounds 3 to 7 in place of Compound 2 to give the desired compounds.

Example 10

1-{3-[Bis(4-hydroxyphenyl)methyl]-1-(2-morpholinoethyl)indol-2-ylcarbonyl}-4-(2-chlorophenyl)-piperazine methanesulfonate (Compound 10 methanesulfonate)

$^1$H-NMR(DMSO-$d_6$) δ(ppm): 2.2-2.35 (1H, m), 2.38 (3H, s), 2.55-2.7 (1H, m), 2.7-2.9 (1H, m), 3.0-3.5 (7H, m), 3.6-3.8 (4H, m), 3.85-4.1 (4H, m), 4.25-4.5 (1H, m), 4.5-4.7 (1H, m), 5.68 (1H, s), 6.55-6.75 (4H, m), 6.8-7.1 (7H, m), 7.1-7.35 (3H, m), 7.38 (1H, d, J=7.9Hz), 7.61(1H, d, J=8.4Hz), 9.1-9.35 (2H, br), 9.85-10.1 (1H, br).
IR(KBr tab.): 1612, 1509, 1480, 1445, 1209 $cm^{-1}$
Melting Point: 167-170°C

Example 11

N-Propyl-3-[bis(4-hydroxyphenyl)methyl]-1-(2-dimethylaminoethyl)indole-2-carboxamide methanesulfonate (Compound 11 methanesulfonate)

$^1$H-NMR(DMSO-$d_6$) δ(ppm): 0.89 (3H, t, J=7.3Hz), 1.45-1.6 (2H, m), 2.33 (3H, s), 2.82 (6H, s), 3.15-3.3 (2H, m), 3.4-3.5 (2H, m), 4.45-4.55 (2H, m), 5.73 (1H, s), 6.64 (4H, d, J=7.9 Hz), 6.85-6.95 (2H, m), 6.91 (4H, d, J=7.9 Hz), 7.15-7.25 (1H, m), 7.57 (1H, d, J=8.2Hz), 8.1-8.2 (1H, m), 9.20 (2H, s), 9.7-9.8 (1H, br).

Example 12

N-Isopropyl-3-[bis(4-hydroxyphenyl)methyl]-1-(2-dimethylaminoethyl)indole-2-carboxamide methanesulfonate (Compound 12 methanesulfonate)

$^1$H-NMR(DMSO-$d_6$) δ(ppm): 1.15 (6H, d, J=6.6Hz), 2.32 (3H, s), 2.83 (6H, s), 3.4-3.5 (2H, m), 4.0-4.15 (1H, m), 4.45-4.6 (2H, m), 5.71 (1H, s), 6.65 (4H, d, J=8.0Hz), 6.85-6.95 (2H, m), 6.91 (4H, d, J=8.0Hz), 7.15-7.25 (1H, m), 7.57 (1H, d, J=8.2Hz), 7.86 (1H, d, J=7.2Hz), 9.21 (2H, br), 9.75-9.9 (1H, br).

Example 13

1-{3-[Bis(4-hydroxyphenyl)methyl]-1-(2-dimethylaminoethyl)indol-2-ylcarbonyl)piperidine methanesulfonate (Compound 13 methanesulfonate)

$^1$H-NMR(DMSO-$d_6$) δ(ppm): 1.1-1.3 (2H, m), 1.4-1.6 (4H, m), 2.32 (3H, s), 2.75-2.95 (8H, m), 3.1-3.4 (3H, m), 3.6-3.7 (1H, m), 4.15-4.3 (1H, m), 4.5-4.65 (1H, m), 5.40 (1H, s), 6.5-6.7 (4H, m), 6.8-7.0 (5H, m), 7.05 (1H, d, J=7.9Hz), 7.15-7.25 (1H, m), 7.57 (1H, d, J=8.6Hz), 9.15 (1H, s), 9.20 (1H, s), 9.5-9.7 (1H, br).

Example 14

N-Cycloheptyl-3-[bis (4-hydroxyphenyl)methyl]-1-(2-dimethylaminoethyl)indole-2-carboxamide methanesulfonate (Compound 14 methanesulfonate)

$^1$H-NMR(DMSO-$d_6$) δ(ppm): 1.4-1.7 (10H, m), 1.8-1.95 (2H, m), 2.31 (3H, s), 2.81 (6H, s), 3.4-3.5 (2H, m), 3.9-4.0 (1H, m), 4.45-4.55 (2H, m), 5.69 (1H, s), 6.65 (4H, d, J=8.4Hz), 6.8-6.95 (2H, m), 6.90 (4H, d, J=8.4Hz), 7.15-7.25 (1H, m), 7.56(1H, d, J=8.3Hz), 7.88 (1H, d, J=7.6Hz), 9.20 (2H, s), 9.5-9.7 (1H, br).

In the following examples 15 to 20, substantially the same procedure as in Example 4 was repeated using

corresponding amines in place of propylamine to give the desired compounds.

Example 15

N,N-Diethyl-3-{bis[4-(methoxymethoxy)phenyl]-methyl}-1-(2-dimethylaminoethyl)indole-2-carboxamide (Compound 15)

$^1$H-NMR($CDCl_3$) δ(ppm): 0.78 (3H, t, J=7.1Hz), 1.22 (3H, t, J=7.1Hz), 2.36 (6H, s), 2.6-2.9 (4H, m), 3.05-3.2 (2H, m), 3.43 (3H, s), 3.47 (3H, s), 3.95-4.15 (1H, m), 4.3-4.45 (1H, m), 5.10 (2H, s), 5.14 (2H, s), 5.54 (1H, s), 6.8-7.0 (5H, m), 7.07 (2H, d, J=8.6Hz), 7.15-7.25 (4H, m), 7.36 (1H, d, J=7.9Hz).

Example 16

4-{3-{Bis[4-(methoxymethoxy)phenyl]methyl}-1-(2-dimethylaminoethyl)indol-2-ylcarbonyl}morpholine (Compound 16)

$^1$H-NMR($CDCl_3$) δ(ppm): 2.29 (6H, s), 2.5-2.7 (2H, m), 2.85-3.1 (3H, m), 3.2-3.35 (1H, m), 3.44(3H, s), 3.46 (3H, s), 3.55-3.75 (4H, m), 4.0-4.15 (1H, m), 4.25-4.4(1H, m), 5.11 (2H, s), 5.14 (2H, s), 5.62 (1H, s), 6.8-7.0 (5H, m), 7.0-7.1 (2H, m), 7.15-7.25 (4H, m), 7.32 (1H, d, J=8.2Hz).

Example 17

N-(4-Propylphenyl)-3-{bis[4-(methoxymethoxy)-phenyl]methyl}-1-(2-dimethylaminoethyl)indole-2-carboxamide (Compound 17)

$^1$H-NMR($CDCl_3$) δ(ppm): 0.93 (3H, t, J=7.4Hz), 1.5-1.7 (2H, m), 2.22 (6H, s), 2.56 (2H, t, J=7.1Hz), 2.85-2.95 (2H, m), 3.46 (6H, s), 4.45-4.55 (2H, m), 5.14 (4H, s), 6.11 (1H, s), 6.85-7.0 (5H, m), 7.0-7.1 (1H, m), 7.1-7.2 (6H, m), 7.2-7.3 (1H, m), 7.3-7.4 (3H, m), 9.25-9.35 (1H, br).

Example 18

N-Cyclooctyl-3-{bis[4-(methoxymethoxy)phenyl]-methyl}-1-(2-dimethylaminoethyl)indole-2-carboxamide (Compound 18)

$^1$H-NMR($CDCl_3$) δ(ppm): 1.35-1.6 (14H, m), 2.28 (6H, s), 2.7-2.9 (2H, m), 3.46 (6H, s), 4.1-4.2 (1H, m), 4.45-4.55 (2H, m), 5.14 (4H, s), 5.94 (1H, s), 6.55-6.65 (1H, m), 6.8-6.9 (2H, m), 6.93 (4H, d, J=8.4Hz), 7.09 (4H, d, J=8.4 Hz), 7.15-7.25 (1H, m), 7.37 (1H, d, J=8.2Hz).

Example 19

1-{3-{Bis[4-(methoxymethoxy)phenyl]methyl}-1-(2-dimethylaminoethyl)indol-2-ylcarbonyl}-4-phenylpiperazine (Compound 19)

$^1$H-NMR($CDCl_3$) δ(ppm): 2.31 (6H, s), 2.35-2.5 (1H, m), 2.6-2.8 (3H, m), 3.0-3.15 (2H, m), 3.15-3.3 (2H, m), 3.42 (3H, s), 3.43 (3H, s), 3.65-3.8 (2H, m), 4.0-4.15 (1H, m), 4.3-4.45 (1H, m), 5.09 (4H, s), 5.64 (1H, s), 6.75-7.0 (8H, m), 7.0-7.25 (8H, m), 7.35 (1H, d, J=8.4Hz).

Example 20

1-Benzyl-4-{3-{bis[4-(methoxymethoxy)phenyl]-methyl}-1-(2-dimethylaminoethyl)indol-2-ylcarbonyl)-piperazine (Compound 20)

$^1$H-NMR($CDCl_3$) δ(ppm): 1.75-1.85 (1H, m), 2.0-2.1 (1H, m), 2.30(6H, s), 2.35-2.45 (2H, m), 2.5-2.8 (2H, m), 2.85-3.0 (1H, m), 3.05-3.2 (1H, m), 3.3-3.45 (2H, m), 3.44 (3H, s), 3.46 (3H, s), 3.6-3.75 (2H, m), 4.0-4.15 (1H, m), 4.25-4.4 (1H, m), 5.11 (2H, s), 5.14 (2H, s), 5.60 (1H, s), 6.8-6.95 (5H, m), 7.0-7.35 (12H, m).

In the following Examples 21 to 22, substantially the same procedure as in Example 2 was repeated using corresponding substituted ethylchloride hydrochlorides in place of 2-dimethylaminoethylchloride hydrochloride to give the desired compounds.

Example 21

1-{3-{Bis[4-(methoxymethoxy)phenyl]methyl}-1-[2-(1-pyrrolidinyl)ethyl]indol-2-ylcarbonyl}-4-(2-chlorophenyl) piperazine (Compound 21)

$^1$H-NMR($CDCl_3$) δ(ppm): 1.8-1.95 (4H, m), 2.15-2.3 (1H, m), 2.5-2.65 (1H, m), 2.65-2.8 (3H, m), 2.8-3.15 (6H, m), 3.2-3.3 (1H, m), 3.41 (3H, s), 3.44 (3H, s), 3.7-3.85 (1H, m), 3.85-4.0 (1H, m), 4.1-4.25 (1H, m), 4.4-4.6 (1H, m), 5.11 (4H, s), 5.67 (1H, s), 6.75-6.85 (1H, m), 6.85-7.0 (6H, m), 7.0-7.25 (7H, m), 7.33 (1H, dd, J=7.9, 1.3Hz), 7.4-7.45 (1H, m).

Example 22

1-{3-{Bis[4-(methoxymethoxy)phenyl]methyl}-1-(2-piperidinoethyl)indol-2-ylcarbonyl}-4-(2-chlorophenyl)-piperazine (Compound 22)

$^1$H-NMR($CDCl_3$) δ(ppm): 1.4-1.5 (2H, m), 1.5-1.65 (4H, m), 2.1-2.2 (1H, m), 2.45-2.55 (4H, m), 2.55-2.7 (2H, m), 2.7-2.9 (2H, m), 2.95-3.2 (2H, m), 3.2-3.35 (1H, m), 3.39 (3H, s), 3.42 (3H, s), 3.65-3.8 (1H, m), 3.95-4.05 (1H, m), 4.05-4.2 (1H, m), 4.3-4.45 (1H, m), 5.10 (4H, s), 5.68 (1H, s), 6.80 (1H, dd, J=7.9, 1.3Hz), 6.85-7.05 (6H, m), 7.05-7.25 (7H, m), 7.32 (1H, dd, J=7.9, 1.3Hz), 7.37 (1H, d, J=8.2Hz).

Example 23

1-{3-{Bis[4-(methoxymethoxy)phenyl]methyl}-1-(3-dimethylaminopropyl)indol-2-ylcarbonyl}-4-(2-chlorophenyl)-piperazine (Compound 23)

1-{3-{Bis[4-(methoxymethoxy)phenyl]methyl}-1-(3-chloropropyl)indol-2-ylcarbonyl)-4-(2-chlorophenyl)-piperazine (1.5 g, 2.13 mmol) obtained in Reference Example 4 was dissolved in a mixed solvent of ethanol (30 ml) and methanol (20 ml), and an aqueous solution of dimethylamine (50%, 5 ml) was added thereto. The resulting solution was heated to 50 to 60°C and stirred for a total of 50 hours while an aqueous solution of dimethylamine was added thereto suitably. The solvent was distilled off under reduced pressure, and a saturated aqueous solution of sodium bicarbonate was added thereto followed by extraction with ethyl acetate. The resulting organic layer was washed with a saturated aqueous solution of sodium chloride and dried over magnesium sulfate, and the solvent was distilled off under reduced pressure to give 1.45 g of a crude product. The obtained crude product was purified with silica gel column chromatography (chloroform/methanol = 50/1 - 20/1) to give 1.35 g (yield :89%) of the title compound.

$^1$H-NMR($CDCl_3$) δ(ppm): 2.0-2.25 (4H, m), 2.34 (6H, s), 2.4-2.5 (1H, m), 2.5-2.6 (1H, m), 2.8-3.0 (2H, m), 3.05-3.3 (2H, m), 3.40 (3H, s), 3.44 (3H, s), 3.7-3.8 (1H, m), 3.85-4.1 (2H, m), 4.25-4.4 (1H, m), 5.10 (2H, s), 5.12 (2H, s), 5.68 (1H, s), 6.75-7.0 (7H, m), 7.0-7.3 (7H, m), 7.33 (1H, dd, J=7.9, 1.5Hz), 7.37(1H, d, J=8.4Hz).

Example 24

1-{3-{Bis[4-(methoxymethoxy)phenyl]methyl}-1-(4-dimethylaminobutyl)indol-2-ylcarbonyl}-4-(2-chlorophenyl)-piperazine (Compound 24)

Substantially the same procedure as in Example 23 was repeated using 1-{3-{bis[4-(methoxymethoxy)phenyl]-methyl}-1-(4-chlorobutyl)indol-2-ylcarbonyl}-4-(2-chlorophenyl)piperazine (1.5 g, 2.09 mmol) obtained in Reference Example 5 and an aqueous solution of dimethylamine to give 1.58 g (quantitative) of the title compound.

$^1$H-NMR($CDCl_3$) δ(ppm): 1.5-1.65 (2H, m), 1.75-2.0(2H, m), 2.1-2.2(1H, m), 2.26(6H, s), 2.25-2.35 (2H, m), 2.5-2.6 (1H, m), 2.8-2.9 (1H, m), 2.95-3.15 (2H, m), 3.15-3.25 (1H, m), 3.40 (3H, s), 3.44 (3H, s), 3.65-3.8 (1H, m), 3.9-4.05 (2H, m), 4.2-4.35 (1H, m), 5.05-5.15 (4H, m), 5.68 (1H, s), 6.75-6.85 (1H, m), 6.85-7.0(6H, m), 7.05-7.25 (7H, m), 7.33 (2H, dd, J=7.9, 1.7Hz).

In the following Examples 25 to 28, substantially the same procedure as in Example 23 or 24 was repeated using a compound obtained in Reference Example 4 or 5 and corresponding amines in place of an aqueous solution of dimethylamine to give the desired compounds.

Example 25

1-{3-{Bis[4-(methoxymethoxy)phenyl]methyl}-1-(3-morpholinopropyl)indol-2-ylcarbonyl}-4-(2-chlorophenyl)-piperazine (Compound 25)

$^1$H-NMR($CDCl_3$) δ(ppm): 1.5-1.8 (1H, br), 2.0-2.2 (3H, m), 2.4-2.6 (6H, m), 2.8-2.9 (1H, m), 2.9-3.0 (1H, m), 3.05-3.25 (2H, m), 3.40 (3H, s), 3.44 (3H, s), 3.65-3.85 (5H, m), 3.85-4.0 (1H, m), 4.0-4.15 (1H, m), 4.25-4.4 (1H, m), 5.1-5.25 (4H, m), 5.68 (1H, s), 6.8-6.85 (1H, m), 6.85-7.05 (6H, m), 7.1-7.25 (7H, m), 7.33 (1H, dd, J=7.9, 1.3Hz), 7.37 (1H, d, J=8.2Hz).

Example 26

1-{3-{Bis[4-(methoxymethoxy)phenyl]methyl}-1-(4-morpholinobutyl)indol-2-ylcarbonyl}-4-(2-chlorophenyl)-piperazine (Compound 26)

$^1$H-NMR($CDCl_3$) δ(ppm): 1.45-1.6 (2H, m), 1.7-2.0 (2H, m), 2.05-2.2 (1H, m), 2.3-2.45 (6H, m), 2.5-2.6 (1H, m), 2.75-2.9 (1H, m), 2.95-3.3 (3H, m), 3.40 (3H, s), 3.44 (3H, s), 3.65-3.75 (5H, m), 3.9-4.1 (2H, m), 4.2-4.3 (1H, m), 5.05-5.2 (4H, m), 5.69 (1H, s), 6.75-6.85 (1H, m), 6.85-7.05 (6H, m), 7.05-7.25 (7H, m), 7.3-7.4 (2H, m).

Example 27

1-{3-{Bis[4-(methoxymethoxy)phenyl]methyl}-1-[3-(1-pyrrolidinyl)propyl]indol-2-ylcarbonyl}-4-(2-chlorophenyl) piperazine (Compound 27)

$^1$H-NMR($CDCl_3$) δ(ppm): 1.75-1.9(4H, m), 1.95-2.2 (3H, m), 2.45-2.65 (7H, m), 2.75-2.9 (1H, m), 2.9-3.0 (1H, m), 3.0-3.1 (1H, m), 3.1-3.25 (1H, m), 3.40 (3H, s), 3.44 (3H, s), 3.65-3.75 (1H, m), 3.9-4.15 (2H, m), 4.25-4.4 (1H, m), 5.05-5.15 (4H, m), 5.69 (1H, s), 6.75-6.85 (1H, m), 6.85-7.0 (6H, m), 7.05-7.25 (7H, m), 7.33 (1H, dd, J=7.9, 1.5Hz), 7.38 (1H, d, J=8.2Hz).

Example 28

1-{3-{Bis[4-(methoxymethoxy)phenyl]methyl}-1-[4-(1-pyrrolidinyl)butyl]indol-2-ylcarbonyl}-4-(2-chlorophenyl) piperazine (Compound 28)

$^1$H-NMR($CDCl_3$) δ(ppm): 1.5-1.65 (2H, m), 1.7-2.0 (7H, m), 2.05-2.2 (1H, m), 2.4-2.6 (6H, m), 2.8-2.9 (1H, m), 2.95-3.15 (2H, m), 3.15-3.3 (1H, m), 3.40 (3H, s), 3.44 (3H, s), 3.65-3.75 (1H, m), 3.9-4.1 (2H, m), 4.2-4.35 (1H, m), 5.05-5.15 (4H, m), 5.68 (1H, s), 6.80(1H, dd, J=7.9, 1.3Hz), 6.85-7.05 (6H, m), 7.05-7.25 (7H, m), 7.3-7.4 (2H, m).

Example 29

1-{3-[Bis(4-methoxyphenyl)methyl]indol-2-ylcarbonyl}-4-(2-chlorophenyl)piperazine (Compound 29)

Substantially the same procedure as in Example 1 was repeated using 4,4'-dimethoxybenzhydrol (2.37 g, 9.71 mmol) and 1-(2-chlorophenyl)-4-(indol-2-ylcarbonyl)-piperazine (3.0 g, 8.83 mmol) to give 4.0 g (yield: 81%) of the title compound.

$^1$H-NMR($CDCl_3$) δ(ppm): 2.65-2.85 (4H, m), 3.5-3.65 (4H, m), 3.75 (6H, s), 5.79 (1H, s), 6.75-6.9 (1H, m), 6.79 (4H, d, J=8.6Hz), 6.9-7.05 (2H, m), 7.1-7.25 (3H, m), 7.17 (4H, d, J=8.6Hz), 7.3-7.4 (2H, m), 8.48 (1H, s).

Example 30

1-{3-[Bis(4-methoxyphenyl)methyl]-1-(2-dimethylaminoethyl)indol-2-ylcarbonyl}-4-(2-chlorophenyl)-piperazine (Compound 30)

Substantially the same procedure as in Example 2 was repeated using Compound 29 (1.0 g, 1.77 mmol) obtained in Example 29 and 2-dimethylaminoethylchloride hydrochloride (266 mg, 1.85 mmol) to give 0.75 g (yield: 67 %) of the title compound.

$^1$H-NMR($CDCl_3$) δ(ppm): 2.1-2.2 (1H, m), 2.3-2.45 (1H, m), 2.40 (6H, s), 2.45-2.6 (1H, m), 2.65-2.9 (2H, m), 3.0-3.15 (2H, m), 3.15-3.3 (1H, m), 3.7-3.85 (1H, m), 3.75 (3H, s), 3.76 (3H, s), 3.9-4.05 (1H, m), 4.05-4.2 (1H, m), 4.4-4.55 (1H, m), 5.67 (1H, s), 6.7-6.9 (6H, m), 6.9-7.05 (2H, m), 7.05-7.15 (2H, m), 7.15-7.3 (4H, m), 7.33 (1H, dd, J=7.9, 1.5Hz), 7.39 (1H, d, J=8.4Hz).

In the following Examples 31 to 32, substantially the same procedure as in Example 30 was repeated using corresponding substituted aminoethylchloride hydrochlorides in place of 2-dimethylaminochloride hydrochloride to give the desired compounds.

Example 31

1-{3-[Bis(4-methoxyphenyl)methyl]-1-(2-morpholinoethyl)indol-2-ylcarbonyl}-4-(2-chlorophenyl)-piperazine (Compound 31)

$^1$H-NMR($CDCl_3$) δ(ppm): 2.05-2.2 (1H, m), 2.45-2.6 (5H, m), 2.6-2.75 (1H, m), 2.75-2.9 (2H, m), 2.95-3.2 (2H, m), 3.2-3.3 (1H, m), 3.6-3.8 (5H, m), 3.75 (6H, s), 3.95-4.2 (2H, m), 4.3-4.45 (1H, m), 5.69(1H, s), 6.7-6.85 (5H, m), 6.9-7.05 (2H, m), 7.05-7.15 (3H, m), 7.15-7.3 (4H, m), 7.3-7.4 (2H, m).

Example 32

1-{3-[Bis(4-methoxyphenyl)methyl]-1-[2-(1-pyrrolidinyl)ethyl]indol-2-ylcarbonyl}-4-(2-chlorophenyl)-piperazine (Compound 32)

$^1$H-NMR($CDCl_3$) δ(ppm): 1.55-1.7 (2H, m), 1.75-1.9 (3H, m), 2.05-2.15 (1H, m), 2.5-2.7 (4H, m), 2.7-2.9 (2H, m), 2.9-3.3 (4H, m), 3.6-3.8 (1H, m), 3.75 (6H, s), 4.0-4.2 (2H, m), 4.3-4.45 (1H, m), 5.69 (1H, s), 6.75 (4H, d, J=8.9 Hz), 6.82 (1H, d, J=8.6 Hz), 6.9-7.0 (2H, m), 7.05-7.15 (3H, m), 7.15-7.25 (4H, m), 7.3-7.4 (2H, m).

In the following Examples 33 to 46, substantially the same procedure as in Example 9 was repeated using corresponding Compound 15 to Compound 28 in place of Compound 2 to give the desired compounds.

Example 33

N,N-Diethyl-3-[bis(4-hydroxyphenyl)methyl]-1-(2-dimethylaminoethyl)indole-2-carboxamide methanesulfonate (Compound 33 methanesulfonate)

$^1$H-NMR(DMSO-$d_6$) δ(ppm): 0.80 (3H, t, J=6.9 Hz), 1.18 (3H, t, J=6.9 Hz), 2.32 (3H, s), 2.7-2.9 (6H, m), 3.1-3.3 (1H, m), 3.3-3.5 (4H, m), 3.5-3.7 (1H, m), 4.1-4.3 (1H, m), 4.5-4.7 (1H, m), 5.33 (1H, s), 6.60 (2H, d, J=8.4Hz), 6.66 (2H, d, J=8.4Hz), 6.84 (2H, d, J=8.4Hz), 6.9-7.0 (3H, m), 7.05 (1H, d, J=7.9Hz), 7.20 (1H, t, J=7.6Hz), 7.58 (1H, d, J=8.2Hz), 9.16 (1H, s), 9.24 (1H, s), 9.55-9.65 (1H, br).
IR(KBr tab.): 3420, 1610, 1590, 1512, 1450, 1202 $cm^{-1}$
Melting Point: 186-187°C

Example 34

4-{3-[Bis(4-hydroxyphenyl)methyl]-1-(2-dimethylaminoethyl)indol-2-ylcarbonyl}morpholine methanesulfonate (Compound 34 methanesulfonate)

$^1$H-NMR(DMSO-$d_6$) δ(ppm): 2.33 (3H, s), 2.86 (6H, br), 2.95-3.05 (1H, m), 3.05-3.2 (1H, m), 3.2-3.5 (4H, m), 3.5-3.7 (4H, m), 4.2-4.35 (1H, m), 4.5-4.65 (1H, m), 5.45 (1H, s), 6.55-6.75 (4H, m), 6.8-7.1 (6H, m), 7.22 (1H, t, J=7.6Hz), 7.58 (1H, d, J=8.6Hz), 9.20 (1H, s), 9.25 (1H, s), 9.55-9.65 (1H, br).
IR(KBr tab.): 3400, 1611, 1513, 1217 $cm^{-1}$
Melting Point: 206-208°C

Example 35

N-(4-Propylphenyl)-3-[bis(4-hydroxyphenyl)-methyl]-1-(2-dimethylaminoethyl)indole-2-carboxamide - methanesulfonate (Compound 35 methanesulfonate)

$^1$H-NMR(DMSO-$d_6$) δ(ppm): 0.90 (3H, t, J=7.3Hz), 1.5-1.7 (2H, m), 2.32 (3H, s), 2.54 (2H, t, J=7.3Hz), 2.83 (6H, s), 3.35-3.5 (2H, m), 4.5-4.65 (2H, m), 5.77 (1H, s), 6.63 (4H, d, J=8.2Hz), 6.9-7.1 (6H, m), 7.1-7.3 (3H, m), 7.45-7.55 (2H, m), 7.61 (1H, d, J=7.9Hz), 9.20 (2H, s), 9.55-9.7 (1H, br), 10.16 (1H, s).
IR(KBr tab.): 3400, 1612, 1513, 1206 $cm^{-1}$
Melting Point: 188-189°C

Example 36

N-Cyclooctyl-3-[bis(4-hydroxyphenyl)methyl]-1-(2-dimethylaminoethyl)indole-2-carboxamide methanesulfonate (Compound 36 methanesulfonate)

$^{1}$H-NMR(DMSO-$d_6$) δ(ppm): 1.4-1.9 (14H, m), 2.32 (3H, s), 2.82 (6H, s), 3.35-3.5 (2H, m), 3.9-4.1 (1H, m), 4.45-4.6 (2H, m), 5.69 (1H, s), 6.65 (4H, d, J=7.9Hz), 6.85-6.95 (6H, m), 7.15-7.25 (1H, m), 7.57 (1H, d, J=8.6Hz), 7.90 (1H, d, J=7.9Hz), 9.23 (2H, s), 9.6-9.7 (1H, br).
IR(KBr tab.): 3380, 3250, 1645, 1613, 1538, 1511, 1225 $cm^{-1}$
Melting Point: 268-270°C (decomposition)

Example 37

1-{3-[Bis(4-hydroxyphenyl)methyl]-1-(2-dimethylaminoethyl)indol-2-ylcarbonyl}-4-phenylpiperazine (Compound 37)

$^{1}$H-NMR(DMSO-$d_6$) δ(ppm): 2.18 (6H, s), 2.5-2.65 (3H, m), 2.75-2.9 (1H, m), 3.05-3.35 (4H, m), 3.65-3.8 (2H, m), 4.0-4.15 (1H, m), 4.2-4.35 (1H, m), 5.47 (1H, s), 6.5-6.7 (4H, m), 6.7-6.95 (6H, m), 6.95-7.25 (6H, m), 7.43 (1H, d, J=8.4Hz), 9.07 (1H, s), 9.15 (1H, s).

Example 38

1-Benzyl-4-{3-[bis(4-hydroxyphenyl)methyl]-1-(2-dimethylaminoethyl)indol-2-ylcarbonyl}piperazine (Compound 38)

$^{1}$H-NMR(DMSO-$d_6$) δ(ppm): 1.75-1.9 (1H, m), 2.0-2.2 (2H, m), 2.17 (6H, s), 2.25-2.45 (2H, m), 2.9-3.05 (1H, m), 3.05-3.2 (1H, m), 3.3-3.5 (3H, m), 3.5-3.7 (2H, m), 3.95-4.1 (1H, m), 4.2-4.35 (1H, m), 5.42 (1H, s), 6.55-6.7 (4H, m), 6.8-6.9 (3H, m), 6.9-7.05 (3H, m), 7.05-7.15 (1H, m), 7.15-7.35 (5H, m), 7.42 (1H, d, J=7.9Hz), 9.10 (1H, s), 9.17 (1H, s) .

Example 39

1-{3-[Bis(4-hydroxyphenyl)methyl]-1-[2-(1-pyrrolidinyl)ethyl]indol-2-ylcarbonyl}-4-(2-chlorophenyl)piperazine methanesulfonate (Compound 39 methanesulfonate)

$^{1}$H-NMR(DMSO-$d_6$) δ(ppm): 1.8-1.9 (2H, m), 1.9-2.05 (2H, m), 2.2-2.35 (1H, m), 2.35 (3H, s), 2.55-2.65 (1H, m), 2.75-3.15 (4H, m), 3.25-3.35 (2H, m), 3.45-3.55 (2H, m), 3.55-3.75 (3H, m), 3.8-3.95 (1H, m), 4.2-4.35 (1H, m), 4.5-4.65 (1H, m), 5.49 (1H, s), 6.55-6.75 (4H, m), 6.85-7.1 (8H, m), 7.2-7.35 (2H, m), 7.39 (1H, d, J=8.0Hz), 7.60 (1H, d, J=7.4Hz), 9.18 (1H, s), 9.26 (1H, s), 9.7-9.8 (1H, br).

Example 40

1-{3-[Bis(4-hydroxyphenyl)methyl]-1-(2-piperidinoethyl)indol-2-ylcarbonyl}-4-(2-chlorophenyl) piperazine methanesulfonate (Compound 40 methanesulfonate)

$^{1}$H-NMR(DMSO-$d_6$) δ(ppm): 1.3-1.5 (1H, m), 1.55-1.9 (6H, m), 2.2-2.3 (1H, m), 2.40 (3H, s), 2.55-2.7 (1H, m), 2.7-3.2 (6H, m), 3.35-3.6 (3H, m), 3.6-3.75 (1H, m), 3.8-3.95 (1H, m), 4.3-4.45 (1H, m), 4.55-4.7 (1H, m), 5.49 (1H, s), 6.55-6.7 (4H, m), 6.8-7.1 (8H, m), 7.15-7.35 (2H, m), 7.37 (1H, d, J=7.9Hz), 7.60 (1H, d, J=7.6Hz), 9.23 (1H, s), 9.31 (1H, s), 9.2-9.4 (1H, br).

Example 41

1-{3-[Bis(4-hydroxyphenyl)methyl]-1-(3-dimethylaminopropyl)indol-2-ylcarbonyl}-4-(2-chlorophenyl)-piperazine methanesulfonate (Compound 41 methanesulfonate)

$^1$H-NMR(DMSO-$d_6$) δ(ppm): 2.0-2.25 (3H, m), 2.33 (3H, s), 2.5- 2.65 (1H, m), 2.76 (6H, s), 2.9-3.0 (1H, m), 3.05-3.15 (2H, m), 3.15-3.3 (1H, m), 3.55-3.7 (1H, m), 3.7-3.9 (1H, m), 3.9-4.05 (2H, m), 4.25-4.45 (2H, m), 5.49 (1H, s), 6.6-6.75 (4H, m), 6.85-7.0 (4H, m), 7.0-7.1 (4H, m), 7.15-7.25 (1H, m), 7.25-7.35 (1H, m), 7.39 (1H, dd, J=7.9, 1.3Hz), 7.55 (1H, d, J=7.9Hz), 9.18 (1H, s), 9.25 (1H, s), 9.25-9.35 (1H, br).

Example 42

1-{3-[Bis(4-hydroxyphenyl)methyl]-1-(4-dimethylaminobutyl)indol-2-ylcarbonyl}-4-(2-chlorophenyl)-piperazine methanesulfonate (Compound 42 methanesulfonate)

$^1$H-NMR(DMSO-$d_6$) δ(ppm) : 1.6-1.9 (4H, m), 2.15-2.3 (1H, m), 2.40 (3H, s), 2.80 (6H, s), 2.5-3.3 (7H, m), 3.55-3.75 (1H, m), 3.9-4.1 (2H, m), 4.3-4.5 (1H, m), 5.58 (1H, s), 6.65-6.85 (4H, m), 6.9-7.05 (4H, m), 7.05-7.2 (4H, m), 7.2-7.3 (1H, m), 7.3-7.4 (1H, m), 7.4-7.55 (1H, m), 7.55-7.7 (1H, m), 9.25 (1H, s), 9.32 (1H, s), 9.2-9.4 (1H, br).

Example 43

1-{3-[Bis(4-hydroxyphenyl)methyl]-1-(3-morpholinopropyl)indol-2-ylcarbonyl}-4-(2-chlorophenyl)-piperazine methanesulfonate (Compound 43 methanesulfonate)

$^1$H-NMR(DMSO-$d_6$) δ(ppm): 2.05-2.3 (3H, m), 2.34 (3H, s), 2.55-2.65 (1H, m), 2.7-2.8 (1H, m), 2.9-3.2 (5H, m), 3.2-3.5 (4H, m), 3.55-3.7 (3H, m), 3.85-4.05 (4H, m), 4.25-4.4 (1H, m), 5.49 (1H, s), 6.6-6.75 (4H, m), 6.85-7.0 (4H, m), 7.0-7.1 (4H, m), 7.15-7.25 (1H, m), 7.25-7.35 (1H, m), 7.39 (1H, dd, J=7.9, 1.8Hz), 7.55 (1H, d, J=7.4Hz), 9.17 (1H, s), 9.25 (1H, s), 9.5-9.65 (1H, br).

Example 44

1-{3-[Bis(4-hydroxyphenyl)methyl]-1-(4-morpholinobutyl)indol-2-ylcarbonyl}-4-(2-chlorophenyl)-piperazine methanesulfonate (Compound 44 methanesulfonate)

$^1$H-NMR(DMSO-$d_6$) δ(ppm): 1.55-1.7 (2H, m), 1.7-1.85 (2H, m), 2.1-2.25 (1H, m), 2.33 (3H, s), 2.5-2.65 (1H, m), 2.7-2.8 (1H, m), 2.85-3.15 (5H, m), 3.15-3.25 (1H, m), 3.25-3.4 (4H, m), 3.55-3.7 (3H, m), 3.8-4.0 (3H, m), 4.25-4.4 (1H, m), 5.49 (1H, s), 6.6-6.7 (4H, m), 6.85-6.96 (4H, m), 7.0-7.1 (4H, m), 7.1-7.2 (1H, m), 7.15-7.25 (1H, m), 7.37 (1H, dd, J=7.9, 1.4Hz), 7.53 (1H, d, J=8.2Hz), 9.17 (1H, s), 9.24 (1H, s), 9.4-9.55 (1H, br).

Example 45

1-{3-[Bis(4-hydroxyphenyl)methyl]-1-[3-(1-pyrrolidinyl)propyl]indol-2-ylcarbonyl}-4-(2-chlorophenyl)-piperazine methanesulfonate (Compound 45 methanesulfonate)

$^1$H-NMR(DMSO-$d_6$) δ(ppm): 1.8-2.25 (7H, m), 2.33 (3H, s), 2.5-2.65 (1H, m), 2.7-2.8 (1H, m), 2.9-3.05 (2H, m), 3.05-3.3 (5H, m), 3.4-3.7 (3H, m), 3.75-4.1 (2H, m), 4.25-4.4 (1H, m), 5.49 (1H, s), 6.6-6.75(4H, m), 6.85-7.0 (4H, m), 7.0-7.1 (4H, m), 7.15-7.25 (1H, m), 7.25-7.35 (1H, m), 7.39 (1H, dd, J=7.9, 1.2Hz), 7.56 (1H, d, J=8.6Hz), 9.17 (1H, s), 9.25 (1H, s), 9.4-9.55 (1H, br).

Example 46

1-{3-[Bis(4-hydroxyphenyl)methyl]-1-[4-(1-pyrrolidinyl)butyl]indol-2-ylcarbonyl}-4-(2-chlorophenyl)-piperazine methanesulfonate (Compound 46 methanesulfonate)

$^1$H-NMR(DMSO-$d_6$) δ(ppm): 1.5-1.65 (2H, m), 1.65-2.0 (6H, m), 2.1-2.25 (1H, m), 2.32 (3H, s), 2.5-2.7 (1H, m), 2.7-2.85 (1H, m), 2.85-3.15 (5H, m), 3.15-3.3 (1H, m), 3.35-3.55 (3H, m), 3.55-3.65 (1H, m), 3.8-4.0 (2H, m), 4.25-4.4 (1H, m), 5.50 (1H, s), 6.6-6.75 (4H, m), 6.85-7.0 (4H, m), 7.0-7.1 (4H, m), 7.1-7.2 (1H, m), 7.25-7.35 (1H, m), 7.39 (1H, dd, J=7.9, 1.3Hz), 7.52 (1H, d, J=8.2Hz), 9.16 (1H, s), 9.24 (1H, s), 9.25-9.4 (1H, br).

Reference Example 1

Ethyl 3-{bis[4-(methoxymethoxy)phenyl]methyl}indole-2-carboxylate

Substantially the same procedure as in Example 1 was repeated using 4,4'-bis(methoxymethoxy)benzhydrol (6.22 g, 32.9 mmol) and ethyl indole-2-carboxylate (10.0 g, 32.9 mmol) to give 12.9 g (yield: 89%) of the title compound.

$^1$H-NMR($CDCl_3$) δ(ppm): 1.39 (3H, t, J=7.3Hz), 3.46 (6H, s), 4.39 (2H, q, J=7.3Hz), 5.14 (4H, s), 6.58 (1H, s), 6.85-6.95 (5H, m), 7.03 (1H, d, J=7.3Hz), 7.13 (4H, d, J=8.2Hz), 7.2-7.25 (1H, m), 7.36 (1H, d, J=8.6Hz), 8.80 (1H, s).

Reference Example 2

Ethyl 3-{bis[4-(methoxymethoxy)phenyl]methyl}-1-(2-dimethylaminoethyl)indole-2-carboxylate

Substantially the same procedure as in Example 2 was repeated using ethyl 3-{bis[4-(methoxymethoxy)phenyl]-methyl}indole-2-carboxylate (12.9 g, 27.0 mmol) obtained in Reference Example 1 and 2-dimethylaminoethylchloride hydrochloride (3.98 g, 27.6 mmol) to give 16.4 g (quantitative) of the title compound as a crude product, which was used as a starting material for Reference Example 3 without further purification.

$^1$H-NMR($CDCl_3$) δ(ppm): 1.37 (3H, t, J=7.2Hz), 2.34 (6H, s), 2.6-2.75 (2H, m), 3.45 (6H, s), 4.35 (2H, q, J=7.2Hz), 4.5-4.65 (2H, m), 5.13 (4H, s), 6.47 (1H, s), 6.8-7.05 (6H, m), 7.09 (4H, d, J=8.5Hz), 7.1-7.2 (1H, m), 7.39 (1H, d, J=8.6Hz).

Reference Example 3

3-{Bis[4-(methoxymethoxy)phenyl]methyl}-1-(2-dimethylaminoethyl)indole-2-carboxylic acid

The crude ethyl 3-{bis[4-(methoxymethoxy)phenyl]-methyl}-1-(2-dimethylaminoethyl)indole-2-carboxylate (16.4 g, approximately 27.0 mmol) obtained in Reference Example 2 was dissolved in 100 ml of ethanol, and a 2N aqueous sodium hydroxide solution (40 ml) was added thereto, followed by heating under reflux for 3 hours. The solvent was distilled off under reduced pressure, water was added to dissolve the residue, and the pH of the solution was adjusted to 5 by adding 2N hydrochloric acid, followed by extraction with chloroform. The resulting organic layer was washed with water and a saturated aqueous solution of sodium chloride, and dried over magnesium sulfate, and the solvent was distilled off under reduced pressure to give 14.4 g of a crude product. The obtained crude product was crystallized from ethyl acetate to give 8.9 g (yield: 64%) of the title compound.

$^1$H-NMR($CDCl_3$) δ(ppm): 2.39 (6H, s), 3.18 (2H, t, J=6.0Hz), 3.45 (6H, s), 4.74 (2H, t, J=6.0Hz), 5.12 (4H, s), 6.68 (1H, s), 6.88 (4H, d, J=8.9Hz), 6.9-7.0 (1H, m), 7.15-7.25 (3H, m), 7.18 (4H, d, J=8.9Hz).

Reference Example 4

1-{3-{Bis[4-(methoxymethoxy)phenyl]methyl}-1-(3-chloropropyl)indol-2-ylcarbonyl}-4-(2-chlorophenyl)-piperazine

To a solution of Compound 1 (5.0 g, 7.99 mmol) obtained in Example 1 in 50 ml of N,N-dimethylformamide was portionwise added sodium hydride (60% in oil, 351 mg, 8.78 mmol) with stirring at room temperature, and 1-bromo-3-chloropropane (0.87 ml, 8.78 mmol) was added thereto, followed by stirring at room temperature for 4 hours. 1-Bromo-3-chloropropane (0.16 ml, 1.62 mmol) was added thereto and the mixture was stirred further for 4.5 hours. The reaction mixture was neutralized with a saturated aqueous solution of ammonium chloride, and water was added thereto followed by extraction with ethyl acetate. The resulting organic layer was washed with water and a saturated aqueous solution of sodium chloride and dried over magnesium sulfate, and the solvent was distilled off under reduced pressure to give 6.5 g of a crude product. The obtained crude product was purified with silica gel column chromatography (ethyl acetate/hexane = 1/3 - 1/1) to give 5.35 g (yield: 95%) of the title compound.

$^1$H-NMR($CDCl_3$) δ(ppm): 2.1-2.35 (3H, m), 2.5-2.65 (1H, m), 2.8-2.9 (1H, m), 2.9-3.15 (2H, m), 3.15-3.3 (1H, m), 3.40 (3H, s), 3.43 (3H, s), 3.45-3.65 (2H, m), 3.65-3.8 (1H, m), 3.9-4.05 (1H, m), 4.0-4.2 (1H, m), 4.4-4.6 (1H, m), 5.05-5.15 (4H, m), 5.69 (1H, s), 6.8-7.05 (7H, m), 7.15-7.3 (7H, m), 7.33 (1H, dd, J=7.9, 1.7Hz), 7.40 (1H, d, J=8.2Hz).

Reference Example 5

1-{3-{Bis[4-(methoxymethoxy)phenyl]methyl}-1-(4-chlorobutyl)indol-2-ylcarbonyl}-4-(2-chlorophenyl)piperazine

Substantially the same procedure as in Reference Example 4 was repeated using Compound 1 (5.0 g, 7.99 mmol) and 1-bromo-4-chlorobutane (1.0 ml, 8.78 mmol) to give 5.3 g (yield: 93%) of the title compound.

$^1$H-NMR($CDCl_3$) δ(ppm): 1.75-1.85 (2H, m), 1.9-2.1 (2H, m), 2.1-2.2 (1H, m), 2.5-2.6 (1H, m), 2.8-2.9 (1H, m), 2.9-3.15 (2H, m), 3.15-3.3 (1H, m), 3.40 (3H, s), 3.43 (3H, s), 3.5-3.6 (2H, m), 3.7-3.85 (1H, m), 3.9-4.05 (2H, m), 4.2-4.35 (1H, m), 5.05-5.15 (4H, m), 5.68 (1H, s), 6.75-7.0 (7H, m), 7.0-7.25 (7H, m), 7.3-7.4 (2H, m).

Industrial Applicability

According to the present invention, there can be provided indole derivatives which are useful as therapeutic agents for osteoporosis.

**Claims**

**1.** An indole derivative represented by formula (I):

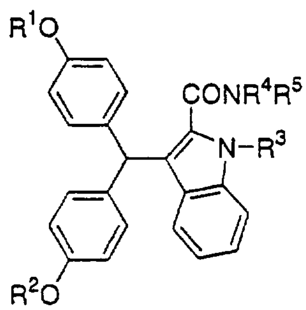

(I)

wherein $R^1$ and $R^2$ independently represent hydrogen, $C_1$-$C_8$ alkyl or $-(CH_2)_n-OR^6$ (wherein $R^6$ represents hydrogen or $C_1$-$C_8$ alkyl, and n is an integer of 1 to 6),
$R^3$ represents hydrogen, $C_1$-$C_8$ alkyl, or

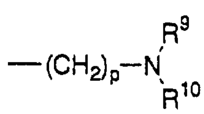

(wherein $R^9$ and $R^{10}$ independently represent hydrogen or $C_1$-$C_8$ alkyl,
or $R^9$ and $R^{10}$ are combined together with the adjacent nitrogen atom to form an alicyclic heterocyclic group

selected from the group consisting of pyrrolidinyl, imidazolidinyl, pyrazolidinyl, piperidino, piperazinyl, homopiperazinyl, morpholino and thiomorpholino (said alicyclic heterocyclic group is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di ($C_1$-$C_8$ alkyl)amino, trifluoromethyl, phenyl (said phenyl is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoy, benzoyl, naphthoyl, halogen, nitro, amino, mono- or di ($C_1$-$C_8$ alkyl)amino and trifluoromethyl), pyridyl and pyrimidinyl), and p represents an integer of 2 to 6),

$R^4$ and $R^5$ independently represent hydrogen, $C_1$-$C_8$ alkyl, $C_3$-$C_8$ alicyclic alkyl, phenyl or naphthyl (said phenyl or naphthyl is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di ($C_1$-$C_8$ alkyl)amino, trifluoromethyl, phenyl (said phenyl is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di ($C_1$-$C_8$ alkyl)amino and trifluoromethyl), pyridyl and pyrimidinyl), or a heterocyclic group selected from the group consisting of pyrrolidinyl, imidazolidinyl, pyrazolidinyl, piperidyl, piperidino, piperazinyl, homopiperazinyl, morpholino and thiomorpholino (said heterocyclic group is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di ($C_1$-$C_8$ alkyl)amino, trifluoromethyl, phenyl (said phenyl is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di ($C_1$-$C_8$ alkyl)amino and trifluoromethyl), pyridyl and pyrimidinyl),

or $R^4$ and $R^5$ are combined together with the adjacent nitrogen atom to form an alicyclic heterocyclic group selected from the group consisting of pyrrolidinyl, imidazolidinyl, pyrazolidinyl, piperidino, piperazinyl, homopiperazinyl, morpholino and thiomorpholino (said alicyclic heterocyclic group is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di ($C_1$-$C_8$ alkyl)amino, trifluoromethyl, phenyl (said phenyl is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di ($C_1$-$C_8$ alkyl)amino and trifluoromethyl), pyridyl and pyrimidinyl, or a pharmaceutically acceptable salt thereof.

2. The indole derivative according to claim 1, wherein $R^1$ and $R^2$ represent hydrogen, or a pharmaceutically acceptable salt thereof.

3. The indole derivative according to claim 2, wherein $R^3$ represents

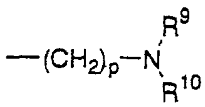

(wherein $R^9$, $R^{10}$ and p have the same meanings as defined above), or a pharmaceutically acceptable salt thereof.

4. The indole derivative according to claim 3, wherein $R^4$ and $R^5$ independently represent hydrogen, $C_1$-$C_8$ alkyl, or $C_3$-$C_8$ alicyclic alkyl, or $R^4$ and $R^5$ are combined together with the adjacent nitrogen atom to form an alicyclic heterocyclic group selected from the group consisting of pyrrolidinyl, imidazolidinyl, pyrazolidinyl, piperidino, piperazinyl, homopiperazinyl, morpholino and thiomorpholino (said alicyclic heterocyclic group is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di ($C_1$-$C_8$ alkyl)amino, trifluoromethyl, phenyl (said phenyl is optionally substituted with 1 to 3 substituents selected from the group consisting of $C_1$-$C_8$ alkyl, hydroxy, $C_1$-$C_8$ alkoxy, $C_1$-$C_8$ alkylthio, $C_7$-$C_{13}$ aralkyl, carboxy, $C_2$-$C_9$ alkoxycarbonyl, $C_1$-$C_7$ alkanoyl, benzoyl, naphthoyl, halogen, nitro, amino, mono- or di ($C_1$-$C_8$ alkyl)amino and trifluoromethyl), pyridyl and pyrimidinyl),

or a pharmaceutically acceptable salt thereof.

**5.** The indole derivative according to claim 1, wherein $R^1$ and $R^2$ represent hydrogen, $R^3$ represents $CH_2CH_2N(CH_3)_2$ and $CONR^4R^5$ represents

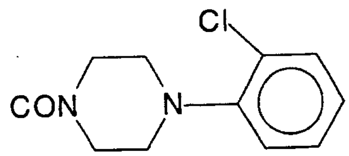

or a pharmaceutically acceptable salt thereof.

**6.** A pharmaceutically acceptable composition comprising a pharmaceutically acceptable carrier and an effective amount of a compound according to claims 1 to 5 as an active ingredient.

**Patentansprüche**

**1.** Indolderivat der Formel (I):

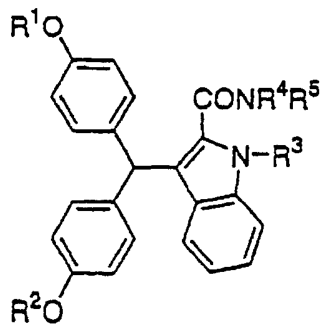

(I)

wobei $R^1$ und $R^2$ unabhängig ein Wasserstoffatom, einen $C_1$-$C_8$-Alkylrest oder einen $-(CH_2)_n-OR^6$-Rest bedeuten (wobei $R^6$ ein Wasserstoffatom oder einen $C_1$-$C_8$-Alkylrest bedeutet, und n eine ganze Zahl von 1 bis 6 bedeutet), $R^3$ ein Wasserstoffatom, einen $C_1$-$C_8$-Alkylrest oder

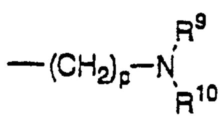

bedeutet (wobei $R^9$ und $R^{10}$ unabhängig voneinander ein Wasserstoffatom oder einen $C_1$-$C_8$-Alkylrest bedeuten, oder $R^9$ und $R^{10}$ zusammen mit dem benachbarten Stickstoffatom verbunden sind, wobei ein alicyclischer heterocyclischer Rest, ausgewählt aus einer Pyrrolidinyl-, Imidazolidinyl-, Pyrazolidinyl-, Piperidino-, Piperazinyl-, Homopiperazinyl-, Morpholino- und Thiomorpholinogruppe erzeugt wird (wobei die alicyclische heterocyclische Gruppe gegebenenfalls mit 1 bis 3 Substituenten, ausgewählt aus $C_1$-$C_8$-Alkylresten, einer Hydroxygruppe, $C_1$-$C_8$-Alkoxy-, $C_1$-$C_8$-Alkylthio-, $C_7$-$C_{13}$-Aralkylresten, einer Carboxygruppe, $C_2$-$C_9$-Alkoxycarbonyl-, $C_1$-$C_7$-Alkanoylresten, einer Benzoyl-, Naphthoylgruppe, Halogenatomen, einer Nitro-, Aminogruppe, Mono- oder Di-($C_1$-$C_8$-alkyl)aminoresten, einer Trifluormethyl-, Phenylgruppe (wobei die Phenylgruppe gegebenenfalls mit 1 bis 3 Substituenten, ausgewählt aus $C_1$-$C_8$-Alkylresten, einer Hydroxygruppe, $C_1$-$C_8$-Alkoxy-, $C_1$-$C_8$-Alkylthio-, $C_7$-$C_{13}$-Aralkylresten, einer Carboxygruppe, $C_2$-$C_9$-Alkoxycarbonyl-, $C_1$-$C_7$-Alkanoylresten, einer Benzoyl-, Naphthoylgruppe, Halogenatomen, einer Nitro-, Aminogruppe, Mono- oder Di-($C_1$-$C_8$alkyl) aminoresten und einer Trifluormethylgruppe substituiert ist), einer Pyridylund Pyrimidinylgruppe substituiert ist, und p eine ganze Zahl von 2 bis 6 bedeutet),

$R^4$ und $R^5$ unabhängig voneinander ein Wasserstoffatom, einen $C_1$-$C_8$-Alkyl-, $C_3$-$C_8$-alicyclischen Alkylrest, eine Phenyl- oder Naphthylgruppe (wobei die Phenyloder Naphthylgruppe gegebenenfalls mit 1 bis 3 Substituenten, ausgewählt aus $C_1$-$C_8$-Alkylresten, einer Hydroxygruppe, $C_1$-$C_8$-Alkoxy-, $C_1$-$C_8$-Alkylthio-, $C_7$-$C_{13}$-Aralkylresten, einer Carboxygruppe, $C_2$-$C_9$-Alkoxycarbonyl-, $C_1$-$C_7$-Alkanoylresten, einer Benzoyl- oder Naphthoylgruppe, Halogenatomen, einer Nitro-, Aminogruppe, Mono- oder Di-($C_1$-$C_8$-alkyl)aminoresten, einer Trifluormethyl-, Phenylgruppe substituiert ist) (wobei die Phenylgruppe gegebenenfalls mit 1 bis 3 Substituenten, ausgewählt aus $C_1$-$C_8$-Alkylresten, einer Hydroxygruppe, $C_1$-$C_8$-Alkoxy-, $C_1$-$C_8$-Alkylthio-, $C_7$-$C_{13}$-Aralkylresten, einer Carboxygruppe, $C_2$-$C_9$-Alkoxycarbonyl-, $C_1$-$C_7$-Alkanoylresten, einer Benzoyl-, Naphthoylgruppe, Halogenatomen, einer Nitro-, Aminogruppe, Mono- oder Di-($C_1$-$C_8$-alkyl)aminoresten und einer Trifluormethylgruppe substituiert ist), einer Pyridyl- und Pyrimidinylgruppe substituiert ist), oder einen heterocyclischen Rest, ausgewählt aus einer Pyrrolidinyl-, Imidazolidinyl-, Pyrazolidinyl-, Piperidyl-, Piperidino-, Piperazinyl-, Homopiperazinyl-, Morpholino- und Thiomorpholinogruppe bedeutet (wobei der heterocyclische Rest gegebenenfalls mit 1 bis 3 Substituenten, ausgewählt aus $C_1$-$C_8$-Alkylresten, einer Hydroxygruppe, $C_1$-$C_8$-Alkoxy-, $C_1$-$C_8$-Alkylthio-, $C_7$-$C_{13}$-Aralkylresten, einer Carboxygruppe, $C_2$-$C_9$-Alkoxycarbonyl-, $C_1$-$C_7$-Alkanoylresten, einer Benzoyl- oder Naphthoylgruppe, Halogenatomen, einer Nitro-, Aminogruppe, Mono- oder Di-($C_1$-$C_8$-alkyl)aminoresten, einer Trifluormethyl-, Phenylgruppe (wobei die Phenylgruppe gegebenenfalls mit 1 bis 3 Substituenten, ausgewählt aus $C_1$-$C_8$-Alkylresten, einer Hydroxygruppe, $C_1$-$C_8$-Alkoxy-, $C_1$-$C_8$-Alkylthio-, $C_7$-$C_{13}$-Aralkylresten, einer Carboxygruppe, $C_2$-$C_9$-Alkoxycarbonyl-, $C_1$-$C_7$-Alkanoylresten, einer Benzoyl-, Naphthoylgruppe, Halogenatomen, einer Nitro-, Aminogruppe, Mono- oder Di-($C_1$-$C_8$alkyl)aminoresten und einer Trifluormethylgruppe substituiert ist), einer Pyridylund Pyrimidinylgruppe substituiert ist), oder $R^4$ und $R^5$ zusammen mit dem benachbarten Stickstoffatom verbunden sind, wobei ein alicyclischer heterocyclischer Rest, ausgewählt aus einer Pyrrolidinyl-, Imidazolidinyl-, Pyrazolidinyl-, Piperidino-, Piperazinyl-, Homopiperazinyl-, Morpholino- und Thiomorpholinogruppe erzeugt wird (wobei der alicyclische heterocyclische Rest gegebenenfalls mit 1 bis 3 Substituenten, ausgewählt aus $C_1$-$C_8$-Alkylresten, einer Hydroxygruppe, $C_1$-$C_8$-Alkoxy-, $C_1$-$C_8$-Alkylthio-, $C_7$-$C_{13}$-Aralkylresten, einer Carboxygruppe, $C_2$-$C_9$-Alkoxycarbonyl-, $C_1$-$C_7$-Alkanoylresten, einer Benzoyl-, Naphthoylgruppe, Halogenatomen, einer Nitro-, Aminogruppe, Mono- oder Di-($C_1$-$C_8$-alkyl)aminoresten, einer Trifluormethyl-, Phenylgruppe (wobei die Phenylgruppe gegebenenfalls mit 1 bis 3 Substituenten, ausgewählt aus $C_1$-$C_8$-Alkylresten, einer Hydroxygruppe, $C_1$-$C_8$-Alkoxy-, $C_1$-$C_8$-Alkylthio-, $C_7$-$C_{13}$-Aralkylgruppen, einer Carboxygruppe, $C_2$-$C_9$-Alkoxycarbonyl-, $C_1$-$C_7$-Alkanoylresten, einer Benzoyl-, Naphthoylgruppe, Halogenatomen, einer Nitro-, Aminogruppe, Mono- oder Di-($C_1$-$C_8$-alkyl)aminoresten und einer Trifluormethylgruppe substituiert ist), einer Pyridyl- und Pyrimidinylgruppe substituiert ist), oder ein pharmazeutisch verträgliches Salz davon.

**2.** Indolderivat nach Anspruch 1, wobei $R^1$ und $R^2$ ein Wasserstoffatom bedeuten oder ein pharmazeutisch verträgliches Salz davon.

**3.** Indolderivat nach Anspruch 2, wobei $R^3$

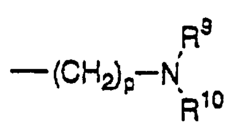

bedeutet (wobei $R^9$, $R^{10}$ und p die vorstehend angegebenen Bedeutungen besitzen) oder ein pharmazeutisch verträgliches Salz davon.

**4.** Indolderivat nach Anspruch 3, wobei $R^4$ und $R^5$ unabhängig voneinander ein Wasserstoffatom, einen $C_1$-$C_8$-Alkylrest oder einen $C_3$-$C_8$-alicyclischen Alkylrest bedeuten, oder $R^4$ und $R^5$ mit dem benachbarten Stickstoffatom verbunden sind, wobei ein alicylischer heterocyclischer Rest, ausgewählt aus Pyrrolidinyl-, Imidazolidinyl-, Pyrazolidinyl-, Piperidino-, Piperazinyl-, Homopiperazinyl-, Morpholinound Thiomorpholinoresten erzeugt wird (wobei der alicyclische heterocyclische Rest gegebenenfalls mit 1 bis 3 Substituenten, ausgewählt aus $C_1$-$C_8$-Alkylresten, einer Hydroxygruppe, $C_1$-$C_8$-Alkoxy-, $C_1$-$C_8$-Alkylthio-, $C_7$-$C_{13}$-Aralkylresten, einer Carboxygruppe, $C_2$-$C_9$-Alkoxycarbonyl-, $C_1$-$C_7$-Alkanoylresten, einer Benzoyl-, Naphthoylgruppe, Halogenatomen, einer Nitro-, Aminogruppe, Mono- oder Di-($C_1$-$C_8$-alkyl)aminoresten, einer Trifluormethyl-, Phenylgruppe (wobei die Phenylgruppe gegebenenfalls mit 1 bis 3 Substituenten, ausgewählt aus $C_1$-$C_8$-Alkylresten, einer Hydroxygruppe, $C_1$-$C_8$-Alkoxy-, $C_1$-$C_8$-Alkylthio-, $C_7$-$C_{13}$-Aralkylresten, einer Carboxygruppe, $C_2$-$C_9$-Alkoxycarbonyl-, $C_1$-$C_7$-Alkanoylresten, einer Benzoyl-, Naphthoylgruppe, Halogenatomen, einer Nitro-, Aminogruppe, Monooder Di-($C_1$-$C_8$-alkyl)aminoresten und

einer Trifluormethylgruppe substituiert ist), einer Pyridyl- und Pyrimidinylgruppe substituiert ist), oder ein pharmazeutisch verträgliches Salz davon.

**5.** Indolderivat nach Anspruch 1, wobei $R^1$ und $R^2$ ein Wasserstoffatom bedeuten, $R^3$ einen Rest $CH_2CH_2N(CH_3)_2$ bedeutet und $CONR^4R^5$

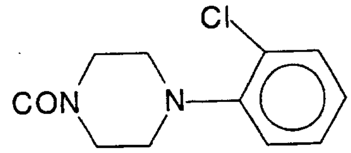

bedeutet oder ein pharmazeutisch verträgliches Salz davon.

**6.** Pharmazeutisch verträgliche Zusammensetzung, umfassend einen pharmazeutisch verträglichen Träger und eine wirksame Menge einer Verbindung nach einem der Ansprüche 1 bis 5 als Wirkstoff.

**Revendications**

**1.** Dérivé d'indole, représenté par la formule (1):

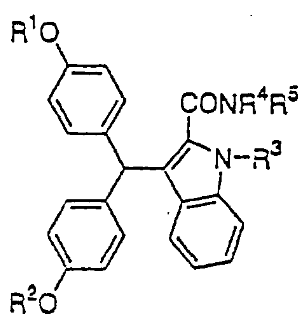

(I)

dans laquelle

$R^1$ et $R^2$ représentent chacun, indépendamment, un atome d'hydrogène ou un groupe de formule

$$-(CH_2)_n-OR^6$$

où $R^6$ représente un atome d'hydrogène ou un groupe alkyle en $C_{1-8}$ et n représente un nombre entier valant de 1 à 6 ;
$R^3$ représente un atome d'hydrogène, un groupe alkyle en $C_{1-8}$ ou un groupe de formule

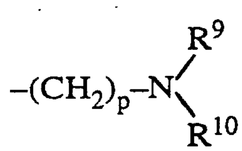

où $R^9$ et $R^{10}$ représentent chacun, indépendamment, un atome d'hydrogène ou un groupe alkyle en $C_{1-8}$, ou bien $R^9$ et $R^{10}$ représentent ensemble, conjointement avec l'atome d'azote adjacent, un groupe hétéroalicyclique choisi dans l'ensemble que constituent les groupes pyrrolidinyle, imidazolidinyle, pyrazolidinyle, pipéridino, pipérazinyle, homopipérazinyle, morpholino et thiomorpholino [lequel groupe hétéroalicyclique peut

porter de 1 à 3 substituants choisis dans l'ensemble constitué par les atomes d'halogène et les groupes alkyle en $C_{1-8}$, hydroxy, alcoxy en $C_{1-8}$, alkylthio en $C_{1-8}$, aralkyle en $C_{7-13}$, carboxy, alcoxycarbonyle en $C_{2-9}$, alcanoyle en $C_{1-7}$, benzoyle, naphtoyle, nitro, amino, mono(alkyle en $C_{1-8}$)amino, di(alkyle en $C_{1-8}$)amino, trifluorométhyle, phényle (lequel groupe phényle peut porter de 1 à 3 substituants choisis dans l'ensemble constitué par les atomes d'halogène et les groupes alkyle en $C_{1-8}$, hydroxy, alcoxy en $C_{1-8}$, alkylthio en $C_{1-8}$, aralkyle en $C_{7-13}$, carboxy, alcoxycarbonyle en $C_{2-9}$, alcanoyle en $C_{1-7}$, benzoyle, naphtoyle, nitro, amino, mono(alkyle en $C_{1-8}$)amino, di(alkyle en $C_{1-8}$)amino et trifluorométhyle), pyridyle et pyrimidinyle], et p représente un nombre entier valant de 2 à 6 ;

$R^4$ et $R^5$ représentent chacun, indépendamment, un atome d'hydrogène ou un groupe alkyle en $C_{1-8}$, cycloalkyle en $C_{3-8}$, phényle ou naphtyle [lesquels groupes phényle et naphtyle peuvent porter de 1 à 3 substituants choisis dans l'ensemble constitué par les atomes d'halogène et les groupes alkyle en $C_{1-8}$, hydroxy, alcoxy en $C_{1-8}$, alkylthio en $C_{1-8}$, aralkyle en $C_{7-13}$, carboxy, alcoxycarbonyle en $C_{2-9}$, alcanoyle en $C_{1-7}$, benzoyle, naphtoyle, nitro, amino, mono(alkyle en $C_{1-8}$)amino, di(alkyle en $C_{1-8}$)amino, trifluorométhyle, phényle (lequel groupe phényle peut porter de 1 à 3 substituants choisis dans l'ensemble constitué par les atomes d'halogène et les groupes alkyle en $C_{1-8}$, hydroxy, alcoxy en $C_{1-8}$, alkylthio en $C_{1-8}$, aralkyle en $C_{7-13}$, carboxy, alcoxycarbonyle en $C_{2-9}$, alcanoyle en $C_{1-7}$, benzoyle, naphtoyle, nitro, amino, mono(alkyle en $C_{1-8}$)amino, di(alkyle en $C_{1-8}$)amino et trifluorométhyle), pyridyle et pyrimidinyle], ou encore un groupe hétérocyclique choisi dans l'ensemble que constituent les groupes pyrrolidinyle, imidazolidinyle, pyrazolidinyle, pipéridyle, pipéridino, pipérazinyle, homopipérazinyle, morpholino et thiomorpholino [lequel groupe hétérocyclique peut porter de 1 à 3 substituants choisis dans l'ensemble constitué par les atomes d'halogène et les groupes alkyle en $C_{1-8}$, hydroxy, alcoxy en $C_{1-8}$, alkylthio en $C_{1-8}$, aralkyle en $C_{7-13}$, carboxy, alcoxycarbonyle en $C_{2-9}$, alcanoyle en $C_{1-7}$, benzoyle, naphtoyle, nitro, amino, mono(alkyle en $C_{1-8}$)amino, di(alkyle en $C_{1-8}$)amino, trifluorométhyle, phényle (lequel groupe phényle peut porter de 1 à 3 substituants choisis dans l'ensemble constitué par les atomes d'halogène et les groupes alkyle en $C_{1-8}$, hydroxy, alcoxy en $C_{1-8}$, alkylthio en $C_{1-8}$, aralkyle en $C_{7-13}$, carboxy, alcoxycarbonyle en $C_{2-9}$, alcanoyle en $C_{1-7}$, benzoyle, naphtoyle, nitro, amino, mono(alkyle en $C_{1-8}$)amino, di (alkyle en $C_{1-8}$)-amino et trifluorométhyle), pyridyle et pyrimidinyle],

ou bien $R^4$ et $R^5$ représentent ensemble, conjointement avec l'atome d'azote adjacent, un groupe hétéroalicyclique choisi dans l'ensemble que constituent les groupes pyrrolidinyle, imidazolidinyle, pyrazolidinyle, pipéridino, pipérazinyle, homopipérazinyle, morpholino et thiomorpholino [lequel groupe hétéroalicyclique peut porter de 1 à 3 substituants choisis dans l'ensemble constitué par les atomes d'halogène et les groupes alkyle en $C_{1-8}$, hydroxy, alcoxy en $C_{1-8}$, alkylthio en $C_{1-8}$, aralkyle en $C_{7-13}$, carboxy, alcoxycarbonyle en $C_{2-9}$, alcanoyle en $C_{1-7}$, benzoyle, naphtoyle, nitro, amino, mono(alkyle en $C_{1-8}$)amino, di(alkyle en $C_{1-8}$)amino, trifluorométhyle, phényle (lequel groupe phényle peut porter de 1 à 3 substituants choisis dans l'ensemble constitué par les atomes d'halogène et les groupes alkyle en $C_{1-8}$, hydroxy, alcoxy en $C_{1-8}$, alkylthio en $C_{1-8}$, aralkyle en $C_{7-13}$, carboxy, alcoxycarbonyle en $C_{2-9}$, alcanoyle en $C_{1-7}$, benzoyle, naphtoyle, nitro, amino, mono(alkyle en $C_{1-8}$)amino, di(alkyle en $C_{1-8}$)amino et trifluorométhyle), pyridyle et pyrimidinyle] ;

ou sel d'un tel dérivé, admissible en pharmacie.

**2.** Dérivé d'indole, conforme à la revendication 1, dans lequel $R^1$ et $R^2$ représentent chacun un atome d'hydrogène, ou un sel d'un tel dérivé, admissible en pharmacie.

**3.** Dérivé d'indole, conforme à la revendication 2, dans lequel $R^3$ représente un groupe de formule

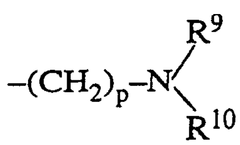

où $R^9$, $R^{10}$ et p ont les significations indiquées plus haut,
ou un sel d'un tel dérivé, admissible en pharmacie.

**4.** Dérivé d'indole, conforme à la revendication 3, dans lequel $R^4$ et $R^5$ représentent chacun, indépendamment, un atome d'hydrogène ou un groupe alkyle en $C_{1-8}$ ou cycloalkyle en $C_{3-8}$, ou bien $R^4$ et $R^5$ représentent ensemble, conjointement avec l'atome d'azote adjacent, un groupe hétéroalicyclique choisi dans l'ensemble que constituent les groupes pyrrolidinyle, imidazolidinyle, pyrazolidinyle, pipéridino, pipérazinyle, homopipérazinyle, morpholino et thiomorpholino [lequel groupe hétéroalicyclique peut porter de 1 à 3 substituants choisis dans l'ensemble constitué par les atomes d'halogène et les groupes alkyle en $C_{1-8}$, hydroxy, alcoxy en $C_{1-8}$, alkylthio en $C_{1-8}$, aralkyle

en $C_{7-13}$, carboxy, alcoxycarbonyle en $C_{2-9}$, alcanoyle en $C_{1-7}$, benzoyle, naphtoyle, nitro, amino, mono(alkyle en $C_{1-8}$)amino, di(alkyle en $C_{1-8}$)amino, trifluorométhyle, phényle (lequel groupe phényle peut porter de 1 à 3 substituants choisis dans l'ensemble constitué par les atomes d'halogène et les groupes alkyle en $C_{1-8}$, hydroxy, alcoxy en $C_{1-8}$, alkylthio en $C_{1-8}$, aralkyle en $C_{7-13}$, carboxy, alcoxycarbonyle en $C_{2-9}$, alcanoyle en $C_{1-7}$, benzoyle, naphtoyle, nitro, amino, mono(alkyle en $C_{1-8}$)amino, di(alkyle en $C_{1-8}$)amino et trifluorométhyle), pyridyle et pyrimidinyle], ou un sel d'un tel dérivé, admissible en pharmacie.

**5.** Dérivé d'indole, conforme à la revendication 1, dans lequel $R^1$ et $R^2$ représentent chacun un atome d'hydrogène, $R^3$ représente le groupe de formule $-CH_2CH_2N(CH_3)_2$, et $CONR^4R^5$ représente le groupe de formule

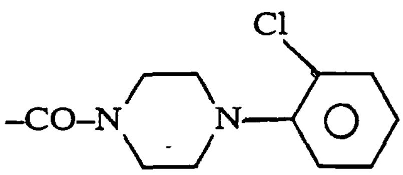

ou un sel d'un tel dérivé, admissible en pharmacie.

**6.** Composition admissible en pharmacie, comprenant un véhicule admissible en pharmacie et, comme ingrédient actif, un composé conforme à l'une des revendications 1 à 5, présent en une quantité suffisante pour qu'il soit efficace.